# EUROPÄISCHE PATENTANMELDUNG

(11) **EP 1 529 521 A2**
(43) Veröffentlichungstag der Anmeldung: **11.05.2005**
(21) Anmeldenummer: 04105522.9
(22) Anmeldetag: 04.11.2004
(51) Int. Cl.: A61K 7/48

(54) **Verwendung von hydrierten Sojaglyceriden in kosmetische Zubereitungen**

(30) Priorität: 10.11.2003 DE 10352371
(71) Anmelder: Beiersdorf AG, 20245 Hamburg (DE)
(72) Erfinder: Max, Heiner, 22529, Hamburg (DE); Bleckmann, Andreas, 22926, Ahrensburg (DE); Riedel, Heidi, 22529, Hamburg (DE); Kunert, Jutta, 22880, Wedel (DE)

(57) **Zusammenfassung**

Verwendung von hydrierten Sojaglyceriden in kosmetischen Zubereitungen.

## Beschreibung

Die vorliegende Erfindung betrifft die Verwendung von hydrierten Sojaglyceriden in kosmetischen Zubereitungen.

Der Wunsch, schön und attraktiv auszusehen, ist von Natur aus im Menschen verwurzelt. Auch wenn das Schönheitsideal im Laufe der Zeit Wandlungen erfahren hat, so ist das Streben nach einem makellosen Äußeren, immer das Ziel der Menschen gewesen. Einen wesentlichen Anteil an einem schönen und attraktiven Äußeren hat dabei der Zustand und das Aussehen der Haut und der Hautanhangsgebilde.

Damit die Haut und die Hautanhangsgebilde, hierzu zählen vor allem die Haare und Nägel, ihre biologischen Funktionen im vollen Umfang erfüllen können, bedürfen sie der regelmäßigen Reinigung und Pflege sowie dem Schutz vor UV-Strahlung. Die Reinigung dient dabei der Entfernung von Schmutz, Schweiß und Resten abgestorbener Körperzellen, die einen idealen Nährboden für Krankheitserreger und Parasiten aller Art bilden. Kosmetische Reinigungsprodukte werden in der Regel in Form von Gelen, Lotionen und Feststoffen (Seifenstücke, Waschsynthets) angeboten. Hautpflegeprodukte, in der Regel Cremes, Salben oder Lotionen, dienen meist der Befeuchtung und Rückfettung der Haut sowie der Versorgung der Haut mit kosmetischen oder dermatologischen Wirkstoffen (z.B. Vitaminen, Antioxidantien, UV-Lichtschutzfiltern). Dies ist gerade dann wichtig, wenn das natürliche Regenerationsvermögen der Haut nicht ausreicht, um die durch Umwelteinflüsse, mechanische oder chemische Reize, Sonnenlicht und Wind entstandenen Belastungen (die u.a. die Hautalterung beschleunigen) und Schäden der Haut auszugleichen.

Hautpflegeprodukte bestehen in der Regel aus Emulsionen. Unter Emulsionen versteht man im allgemeinen heterogene Systeme, die aus zwei nicht oder nur begrenzt miteinander mischbaren Flüssigkeiten bestehen, die üblicherweise als Phasen bezeichnet werden und bei denen eine der beiden Flüssigkeiten in Form feinster Tröpfchen in der anderen Flüssigkeit dispergiert ist. Äußerlich und mit bloßem Auge betrachtet erscheinen Emulsionen homogen.

Sind die beiden Flüssigkeiten Wasser und Öl und liegen Öltröpfchen fein verteilt in Wasser vor, so handelt es sich um eine Öl-in-Wasser-Emulsion (O/W-Emulsion, z. B. Milch). Der Grundcharakter einer O/W-Emulsion ist durch das Wasser geprägt. Bei einer Wasser-in-Öl-Emulsion (W/O-Emulsion, z. B. Butter) handelt es sich um das umgekehrte Prinzip, wobei der Grundcharakter hier durch das Öl bestimmt wird.

Die Ölphase kosmetischer Emulsionen kann aus einer Vielzahl lipophiler Verbindungen, beispielsweise Ölen, Fetten, Wachsen, Silikonölen, aufgebaut werden. Die Öle, Fette und Wachse unterscheiden sich dabei unter anderem in ihrer Polarität. In der Regel wird diese definiert über die Grenzflächenspannung eines Öls gegenüber Wasser. Dabei gilt, dass die Polarität der betreffenden Ölphase umso größer ist, je niedriger die Grenzflächenspannung zwischen dieser Ölphase und Wasser ist.
Die Grenzflächenspannung ist diejenige Kraft, die an einer gedachten, in der Grenzfläche zwischen zwei Phasen befindlichen Linie der Länge von einem Meter wirkt. Die physikalische Einheit für diese Grenzflächenspannung errechnet sich klassisch nach der Beziehung Kraft/Länge und wird gewöhnlich in mN/m (Millinewton geteilt durch Meter) wiedergegeben. Sie hat positives Vorzeichen, wenn sie das Bestreben hat, die Grenzfläche zu verkleinern. Im umgekehrten Falle hat sie negatives Vorzeichen. Als polar werden Lipide angesehen, deren Grenzflächenspannung gegen Wasser weniger als 20 mN/m beträgt als unpolar solche, deren Grenzflächenspannung gegen Wasser mehr als 30 mN/m beträgt. Lipide mit einer Grenzflächenspannung gegen Wasser zwischen 20 und 30 mN/m werden im allgemeinen als mittelpolar bezeichnet.
Polare Öle, sind beispielsweise solche aus der Gruppe der Lecithine und der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen.
Zu den lipophilen Verbindungen mit mittlerer Polarität werden die Verbindungen aus der Gruppe der Ether, Ester und Wachse gezählt, beispielsweise Alkybenzoate.
Zur Gruppe der unpolaren Ölkomponenten zählen beispielsweise alle Kohlenwasserstoff-Verbindungen wie Vaseline (Petrolatum), Paraffinöl, Squalan, Squalen, Polyolefine und hydrogenierte Polyisobutene.
Die Ölkomponenten kosmetischer Zubereitungen können ferner über ihre Herkunft (pflanzlich, tierisch, mineralisch) und ihren chemischen Aufbau (z.B. Ether, Ester, Kohlenwasserstoffe, Silikonöle) sowie anhand ihres Aggregatzustandes (fest, flüssig) unterschieden werden.

Weit verbreitet ist der Einsatz von Wachsen in kosmetischen Zubereitungen. Sie dienen in den Formulierungen meist als Konsistenzgeber. Darüber hinaus verhindern sie, nicht zuletzt wegen ihres guten Adsorbtionsvermögens gegenüber Ölen, das Ausölen der Ölkomponente in W/O-Emulsionen bei höherer Temperatur.
Als Wachse wurden ursprünglich im Pflanzen- und Tierreich weit verbreitete und chemisch weit gefächerte Substanzgemische, deren Hauptkomponenten Ester höherer Fettsäuren mit ebenfalls höheren primären Alkoholen sind, bezeichnet. Heute versteht man im Zusammenhang mit der industriellen Verwertung der Wachse der überwiegenden Substitution dieser Naturstoffe durch mineralische und synthetische Rohstoffe unter Wachse Stoffe und Stoffgemische mit bestimmten verarbeitungstechnologischen Eigenschaften. Solche Stoffe bzw. Stoffgemische sind bei 20 °C knetbar fest bis brüchig hart, grob bis feinkristallin, durchscheinend bis opak, jedoch nicht glasartig, oberhalb 40 °C ohne Zersetzung schmelzend, schon wenig oberhalb des Schmelzpunktes verhältnismäßig niedrigviskos, stark temperaturabhängig in Konsistenz und Löslichkeit. Charakteristisch für Wachse ist, dass sie nicht nur aus einer Vielzahl analoger bzw. ähnlicher chemisch definierter Verbindungen bestehen, sondern vielfach auch Bestandteile mehrerer Stoffklassen nebeneinander enthalten.
Der Einsatz von Wachsen in kosmetischen Zubereitungen (insbesondere Emulsionen) bringt jedoch in der Regel eine Reihe sensorischer Nachteile mit sich, welche die Anwendung der Zubereitungen beim Verbraucher unattraktiv machen. Der Einsatz größerer Mengen von Wachsen mit einem Schmelzpunkt von > 30°C (insbesondere mit einem Schmelzpunkt zwischen 38 und 44 °C) führt in der Regel zu einem trockenen, stumpfen Hautgefühl. Ferner werden derartige Zubereitungen auf der Haut schwer verteilbar.
Es war daher eine Aufgabe der vorliegenden Erfindung, kosmetische und/oder dermatologische Zubereitungen zu entwickeln, die einen Wachsgehalt (insbesondere an Wachsen mit einem Schmelzpunkt von > 30°C) von bis zu 10 Gewichts-%, bezogen auf das Gesamtgewicht der Zubereitung aufweisen können, ohne ein unangenehmes trockenstumpfes Hautgefühl nach der Anwendung zurück zu lassen und die beim Auftragen auf die Haut leicht zu verteilen sind.

Einer der wichtigsten Aufgaben kosmetischer Hautpflegeprodukte besteht darin, die Haut zu befeuchten. Für eine wirksame Befeuchtung der Haut sind dabei zwei Komponenten notwendig. Neben Wasser als eigentlichem Befeuchtungsmittel bedarf es Verbindungen, welche das Wasser in ausreichender Menge in der Haut speichern, fixieren und bei Bedarf an die Hautzellen abgeben. Derartige Verbindungen werden meist als Feuchthaltefaktoren bezeichnet. Zu den natürlichen Feuchthaltefaktoren der Haut (engl. Natural Moisturizing Factor, NMF) zählen beispielsweise Harnstoff und bestimmte Aminosäuren. Die Aufgabe kosmetischer Hautpflegeprodukte besteht nun darin, die Haut einerseits mit Wasser zu versorgen und andererseits die durch die Beanspruchung der Haut verloren gegangenen Feuchtigkeitsfaktoren zu ersetzen. Zu den klassischen Hautbefeuchtungsmitteln zählen in der Kosmetik unter anderem Polyole wie Glycerin und Sorbit. Daneben kommen jedoch auch andere Verbindungen zum Einsatz wie ethoxylierte Polyole und hydrolysierte Proteine. Am weitesten verbreitet ist jedoch das Glycerin.
Ein großer Nachteil am Stand der Technik besteht in den negativen sensorischen Eigenschaften Polyol-haltiger kosmetischer und/oder dermatologischer Zubereitungen. Diese fühlen sich in der Regel auf der Haut klebrig und schmierig an und machen die entsprechenden Produkte für den Verbraucher unattraktiv. Dies ist insbesondere dann der Fall, wenn größere Mengen (größer 5 Gewichts-%) an Polyolen (insbesondere Glycerin) in den Zubereitungen enthalten sind.

Es war daher die Aufgabe der vorliegenden Erfindung die Nachteile des Standes der Technik zu beseitigen beziehungsweise zu vermindern und Polyol-haltige kosmetische und/oder dermatologischer Zubereitungen zu entwickeln, deren klebriges und schmieriges Hautgefühl reduziert ist.
Besonders großer Beliebtheit bei den Verbrauchern erfreuen sich Öl- bzw. Lipidkomponenten pflanzlichen Ursprungs, denen eine besonders gute Verträglichkeit und Umweltfreundlichkeit nachgesagt wird. Leider ist die Herstellung/Gewinnung derartiger Lipidkomponenten relativ aufwendig und kostspielig, so dass Kosmetika auf Basis pflanzlicher Lipide für den Verbraucher in der Regel relativ teuer sind. Auch zeigen Zubereitungen auf Basis pflanzlicher Öle in Kombination mit Wachsen und/oder Polyolen die bereits oben beschriebenen negativen sensorischen Eigenschaften in einer recht intensiven Ausprägung.
Es war daher die Aufgabe der vorliegenden Erfindung, kosmetische und/oder dermatologische Zubereitungen auf der Basis pflanzlicher Lipide zu entwickeln, die besonders kostengünstig sind und selbst mit größeren Mengen an Wachsen und/oder Polyolen kombiniert werden können ohne die bekannten negativen sensorischen Eigenschaften (z.B. klebrig und schmierig bei Einsatz von Polyolen, unangenehmes trocken-stumpfes Hautgefühl sowie schlechte Verteilbarkeit bei Wachsen) aufzuweisen.
Ferner war es die Aufgabe der vorliegenden Erfindung, derartige kosmetische und/oder dermatologische Zubereitungen zu entwickeln, die
- zur Behandlung und Prophylaxe der Symptome der Hautalterung,
- zur Straffung und Glättung der Haut sowie zur Reduzierung von Falten,
- zur Befeuchtung der Haut,
- zur Erhöhung der Hautelastizität und Weichheit der Haut,
- zur Regulierung des Fettgehaltes der Haut und/oder
- zur Erhöhung der Wirksamkeit kosmetischer Wirkstoffe

eingesetzt werden können ohne die bekannten negativen sensorischen Eigenschaften (z.B. klebrig und schmierig bei Einsatz von Polyolen, unangenehmes trocken-stumpfes Hautgefühl sowie schlechte Verteilbarkeit bei Wachsen) aufzuweisen.

Überraschend gelöst werden die Aufgaben durch die Verwendung von hydrierten Sojaglyceriden in kosmetischen Zubereitungen.

Dabei ist erfindungsgemäß insbesondere
- die Verwendung von hydrierten Sojaglyceriden in kosmetischen Zubereitungen zur Behandlung und Prophylaxe der Symptome der Hautalterung,
- die Verwendung von hydrierten Sojaglyceriden in kosmetischen Zubereitungen zur Straffung und Glättung der Haut sowie zur Reduzierung von Falten,
- die Verwendung von hydrierten Sojaglyceriden in kosmetischen Zubereitungen zur Befeuchtung der Haut,
- die Verwendung von hydrierten Sojaglyceriden in kosmetischen Zubereitungen zur Erhöhung der Hautelastizität und Weichheit der Haut,
- die Verwendung von hydrierten Sojaglyceriden in kosmetischen Zubereitungen zur Regulierung des Fettgehaltes der Haut sowie
- die Verwendung von hydrierten Sojaglyceriden in kosmetischen Zubereitungen zur Erhöhung der Wirksamkeit kosmetischer Wirkstoffe.
- die Verwendung von hydrierten Sojaglyceriden in kosmetischen Zubereitungen zur Regeneration der Haut
- die Verwendung von hydrierten Sojaglyceriden in kosmetischen Zubereitungen zur Verfeinerung der Hautextur und Poren
- die Verwendung von hydrierten Sojaglyceriden in kosmetischen Zubereitungen zur Stärkung der Hautbarriere
- die Verwendung von hydrierten Sojaglyceriden in kosmetischen Zubereitungen zur Straffung der Haut
- die Verwendung von hydrierten Sojaglyceriden in kosmetischen Zubereitungen zur Entgiftung der Haut

Die erfindungsgemäß vorteilhaft verwendeten hydrierten Sojaglyceride (beispielsweise CAS Nr. 68201-48-9, INCI Hydrogenated Soy Glycerides) können gewählt werden aus den Mono-, Di- und/oder Triglyceriden, wobei die Triglyceride erfindungsgemäß bevorzugt sind.

Die erfindungsgemäß verwendeten Sojaglyceride enthalten bevorzugt Fettsäureglyceride, die aus Fettsäuren mit einer Kohlenstoffkettenlänge von 18 bis 20 Kohlenstoffatomen bestehen.

Erfindungsgemäß vorteilhaft sind die Fettsäuren der erfindungsgemäß verwendeten Sojaglyceride zu 20 -50% hydriert (gesättigt), bevorzugt zu 25-35% hydriert (gesättigt) und besonders bevorzugt zu 30% hydriert (gesättigt).
Ferner liegen die Fettsäuren der erfindungsgemäß verwendeten Sojaglyceride vorteilhaft zu 50 - 80 %, bevorzugt zu 55 -75% und besonders bevorzugt zu 70% in Form einfach ungesättigter Fettsäuren vor.

Dem entsprechend bedeutet "hydrierte Sojaglyceride" erfindungsgemäß nicht, dass die Sojaglyceride vollständig hydriert sind. Vielmehr ist unter "hydrierte Sojaglyceride" erfindungsgemäß zu verstehen, dass ein (oben näher definierter) ein Anteil der Fettsäuren der Sojaglyceride hydriert sind.

Die erfindungsgemäß verwendeten hydrierten Sojaglyceride werden in den Zubereitungen erfindungsgemäß vorteilhaft in einer Konzentration von 0,5 bis 7 Gewichts-% und erfindungsgemäß bevorzugt in einer Konzentration von 2 bis 5 Gewichts-%, jeweils bezogen auf das Gesamtgewicht der Zubereitung eingesetzt.

Vorteilhaft im Sinne der vorliegenden Erfindung ist insbesondere die Verwendung von hydrierten Sojaglyceriden, wenn die Zubereitung in Form einer Emulsion vorliegt. Die Zubereitungen im Sinne der vorliegenden Erfindung können dann neben einer oder mehrerer Ölphasen zusätzlich eine oder mehrere Wasserphasen enthalten und beispielsweise in Form von W/O-, O/W-, W/O/W- oder O/W/O-Emulsionen (oder entsprechenden Silikonölemulsionen) vorliegen. Solche Formulierungen können vorzugsweise auch eine Mikroemulsion (z. B. eine PIT-Emulsion), eine Feststoff-Emulsionen (d. h. eine Emulsion, welche durch Feststoffe stabilisiert ist, z. B. eine Pickering-Emulsion), eine sprühbare oder schäumbare Emulsion oder eine Hydrodispersion sein. Des Weiteren können die Zubereitungen im Sinne der vorliegenden Erfindung auch nahezu wasserfrei sein (Wassergehalt unter 5 Gewichts-% bezogen auf das Gesamtgewicht der Zubereitung) wie beispielsweise Ölgele. Erfindungsgemäß sind auch feststoffhaltige Emulsionen wie Make-up oder Wundschutzprodukte sowie transparent oder translucente bis milchige gelartige Produkte.

Erfindungsgemäß bevorzugt sind dabei O/W-Emulsionen, Hydrodispersionen, Gelcremes.

Erfindungsgemäß vorteilhafte O/W-Emulgatoren werden erfindungsgemäß vorzugsweise aus der folgenden Gruppe gewählt:
Glycerylstearat in Kombination mit Ceteareth-20, Ceteareth-25, Ceteareth-6 in Kombination mit Stearylalkohol, Cetylstearylalkohol in Kombination mit PEG-40-Ricinusöl und Natriumcetylstearylsulfat, Triceteareth-4 Phosphat, Glycerylstearat, Natriumcetylstearylsulfat, Lecithin Trilaureth-4 Phosphat, Laureth-4 Phosphat, Stearinsäure, Propylenglycolstearat SE, PEG-25-hydriertes Ricinusöl, PEG-54-hydriertes Ricinusöl, PEG-6 Caprylsäure/Caprinsäureglyceride, Glyceryloleat in Kombination mit Propylenglycol, PEG-9-Stearat, PEG-20 Stearat, PEG-30-Stearat, PEG-40-Stearat, PEG-100-Stearat, Ceteth-2, Ceteth-20, Polysorbate-20, Polysorbate-60, Polysorbate-65, Polysorbate-100, Glycerylstearat in Kombination mit PEG-100 Stearat, Glycerylmyristat, Glyceryllaurat, PEG-40-Sorbitanperoleat, Laureth-4, Ceteareth-3, Isostearylglycerylether, Cetylstearylalkohol in Kombination mit Natrium Cetylstearylsulfat, Laureth-23, Steareth-2, Glycerylstearat in Kombination mit PEG-30 Stearat, PEG-40-Stearat, Glycol Distearat, PEG-22-Dodecyl Glycol Copolymer, Polyglyceryl-2-PEG-4-Stearat, Ceteareth-12, Ceteareth-20, Ceteareth-30, Methyl-glucosesesquistearat, Steareth-10, PEG-20-Stearat, Steareth-2 in Kombination mit PEG-8 Distearat, Steareth-21, Steareth-20, Isosteareth-20, PEG-45/ Dodecylglycol-Copolymer, Methoxy-PEG-22/Dodecylglycol-Copolymer, PEG-40-Sorbitanperoleat, PEG-40-Sor-bitanperisostearat, PEG-20-Glycerylstearat, PEG-20-Glycerylstearat, PEG-8-Bienenwachs, Polyglyceryl-2-laurat, Isostearyldiglycerylsuccinat, Stearamidopropyl-PG-dimoniumchloridphosphat, Glycerylstearat SE, Ceteth-20, Triethylcitrat, PEG-20-Methylglucosesesquistearat, Glycerylstearatcitrat, Cetylphosphat, Cetearyl Sulfat, Sorbitansesquioleat, Triceteareth-4-Phosphat, Trilaureth-4-Phosphat, Polyglyceryl-methylglucosedistearat, Kaliumcetylphosphat, Isosteareth-10, Polyglyceryl-2-sesquiisostearat, Ceteth-10, Oleth-20, Isoceteth-20, Glycerylstearat in Kombination mit Ceteareth-20, Ceteareth-12, Cetylstearylalkohol und Cetylpalmitat, Cetylstearylalkohol in Kombination mit PEG-20 Stearat, PEG-30-Stearat, PEG-40-Stearat, PEG-100-Stearat.

Als erfindungsgemäß besonders vorteilhafte O/W-Emulgatoren werden Glycerylstearatcitrat, Cetearyl Sulfat, ethoxylierte Fettsäuren (PEG-40 Stearat, PEG-100 Stearat) und/oder Phosphatemulgatoren (z.B. Cetyl Phosphat) oder Salze der Palmitin- und Stearinsäure eingesetzt.

Als erfindungsgemäß vorteilhafte W/O-Emulgatoren können der oder die W/O-Emulgatoren aus der Gruppe der Silikonemulgatoren gewählt werden. Vorteilhaft aus der Gruppe der Alkylmethiconcopolyole und/oder Alkyl-Dimethiconcopolyole, insbesondere aus der Gruppe der Verbindungen, welche gekennzeichnet sind durch die folgende chemische Struktur: 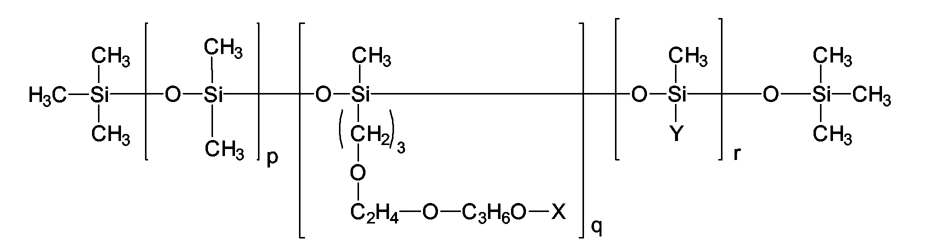 bei welcher X und Y unabhängig voneinander gewählt werden aus der Gruppe H (Wasserstoff) sowie der verzweigten und unverzweigten Alkylgruppen, Acylgruppen und AIkoxygruppen mit 1-24 Kohlenstoffatomen, p eine Zahl von 0 - 200 darstellt, q eine Zahl von 1-40 darstellt, und r eine Zahl von 1-100 darstellt.

Ein Beispiel für besonders vorteilhaft im Sinne der vorliegenden Erfindung zu verwendende Silikonemulgatoren sind Dimethiconcopolyole, welche von der Gesellschaft Th.Goldschmidt AG unter den Warenbezeichnungen ABIL® B 8842, ABIL® B 8843, ABIL® B 8847, ABIL® B 8851, ABIL® B 8852, ABIL® B 8863, ABIL® B 8873 und ABIL® B 88183 verkauft werden.

Ein weiteres Beispiel für besonders vorteilhaft im Sinne der vorliegenden Erfindung zu verwendende grenzflächenaktiven Substanzen ist das Cetyl Dimethiconcopolyol, welches von der Gesellschaft Th.Goldschmidt AG unter der Warenbezeichnung ABIL® EM 90 verkauft wird.

Ein weiteres Beispiel für besonders vorteilhaft im Sinne der vorliegenden Erfindung zu verwendende grenzflächenaktiven Substanzen ist das Cyclomethicon Dimethiconcopolyol, welches von der Gesellschaft Th.Goldschmidt AG unter der Warenbezeichnung ABIL® EM 97 verkauft wird.

Weiterhin hat sich als ganz besonders vorteilhaft der Emulgator Laurylmethiconcopolyol herausgestellt, welcher unter der Warenbezeichnung Dow Corning® 5200 Formulation Aid von der Gesellschaft Dow Corning Ltd. erhältlich ist.

Ein weiterer vorteilhafter Silikonemulgator ist, Octyl Dimethicon Ethoxy Glucosid' der Firma Wacker.

Des Weiteren können der oder die W/O-Emulgatoren erfindungsgemäß vorzugsweise aus der folgenden Gruppe gewählt werden:
Sorbitanstearat, Sorbitanoleat, Lecithin, Glyceryllanolat, Lanolin, Ozokerit, hydriertes Ricinusöl, Glycerylisostearat, Glycerylmono-, distearate, Polyglyceryl-3-Oleat, Wollwachssäuregemische, Wollwachsalkoholgemische, Pentaerythrithylisostearat, Polyglyceryl-3-diisostearat, Methylglucosedioleat, PEG-7-hydriertes Ricinusöl, Polyglyceryl-4-isostearat, Hexyllaurat, Acrylat/ C₁₀₋₃₀-Alkylacrylat-Crosspolymer, Sorbitanisostearat, Poloxamer 101, Polyglyceryl-2-dipolyhydroxystearat, Polyglyceryl-4-dipolyhydroxystearat, PEG-30-dipolyhydroxystearat, Diisostearoylpolyglyceryl-3-diisostearat, Polyglyceryl-3-dipolyhydroxystearat, Polyglyceryl-3-dioleat.

Eine erfindungsgemäße wässrige Phase der erfindungsgemäßen Zubereitungen kann vorteilhaft übliche kosmetische Hilfsstoffe enthalten, wie beispielsweise Alkohole, insbesondere solche niedriger C-Zahl, vorzugsweise Ethanol und/oder Isopropanol, Diole oder Polyole niedriger C-Zahl sowie deren Ether, vorzugsweise Propylenglykol, Glycerin, Butylenglykol, Sorbitol, Ethylenglykol, Ethylenglykolmonoethyl- oder -monobutylether, Propylenglykolmonomethyl, -monoethyl- oder -monobutylether, Diethylenglykolmonomethyl- oder -monoethylether und analoge Produkte.

Erfindungsgemäß vorteilhaft beträgt der Gehalt an einem oder mehreren Polyolen in den Zubereitungen, in denen die hydrierten Sojaglyceride erfindungsgemäß verwendet werden 5 bis 20 Gewichts-% und bevorzugt 7,5 bis 12,5 Gewichts-%, jeweils bezogen auf das Gesamtgewicht der Zubereitung. Erfindungsgemäß besonders bevorzugt wird als Polyol Glycerin eingesetzt.

Ferner kann die erfindungsgemäße wässrige Phase Polymere, Schaumstabilisatoren, Elektrolyte sowie insbesondere ein oder mehrere Verdickungsmittel, welches oder welche vorteilhaft gewählt werden können aus der Gruppe Siliciumdioxid, Aluminiumsilikate, Polysaccharide bzw. deren Derivate, z. B. Hyaluronsäure, Xanthangummi, Hydroxypropylmethylcellulose, besonders vorteilhaft aus der Gruppe der Polyacrylate, bevorzugt ein Polyacrylat aus der Gruppe der sogenannten Carbopole [von der Fa. Bf. Goodrich], beispielsweise Carbopole der Typen 980, 981, 1382, 2984, 5984, ETD 2020, ETD 2050, Ultrez 10, jeweils einzeln oder in Kombination, enthalten.

Eine erfindungsgemäß vorteilhafte Öl-(bzw. Lipid-)phase kann, neben den erfindungsgemäß verwendeten hydrierten Sojaglyceriden vorteilhaft weitere lipophile Verbindungen enthalten.

Besonders vorteilhafte polare Lipide im Sinne der vorliegenden Erfindung sind alle nativen Lipide, wie z. B. Olivenöl, Sonnenblumenöl, Sojaöl, Erdnußöl, Rapsöl, Mandelöl, Palmöl, Kokosöl, Rizinusöl, Weizenkeimöl, Traubenkernöl, Distelöl, Nachtkerzenöl, Macadamianußöl, Maiskeimöl, Avocadoöl und dergleichen sowie die im folgenden aufgelisteten.

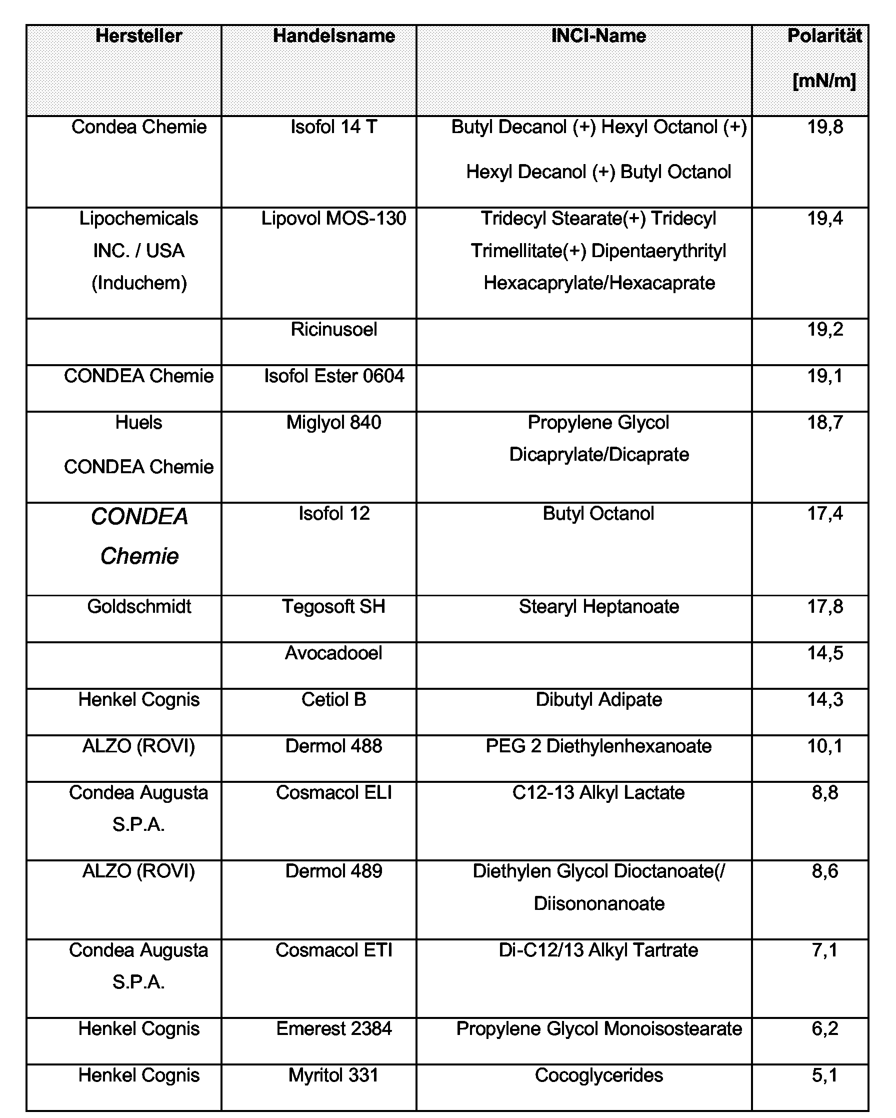

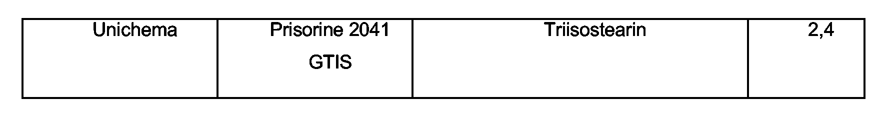

Ferner kann die Ölphase vorteilhaft gewählt werden aus der Gruppe der Dialkylether, der Gruppe der gesättigten oder ungesättigten, verzweigten oder unverzweigten Alkohole. Es ist insbesondere vorteilhaft, wenn die Ölphase einen Gehalt an C₁₂₋₁₅-Alkylbenzoat aufweist oder vollständig aus diesem besteht.

Ferner kann die Ölphase vorteilhaft gewählt werden aus der Gruppe der Guerbetalkohole. Guerbetalkohole sind benannt nach Marcel Guerbet, der ihre Herstellung erstmalig beschrieb. Sie entstehen nach der Reaktionsgleichung 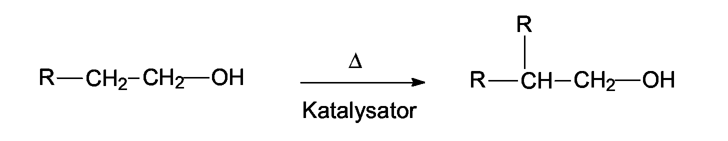 durch Oxidation eines Alkohols zu einem Aldehyd, durch Aldol-Kondensation des Aldehyds, Abspaltung von Wasser aus dem Aldol- und Hydrierung des Allylaldehyds. Guerbetalkohole sind selbst bei niederen Temperaturen flüssig und bewirken praktisch keine Hautreizungen. Vorteilhaft können sie als fettende, überfettende und auch rückfettend wirkende Bestandteile in Haut- und Haarpflegemitteln eingesetzt werden.

Die Verwendung von Guerbet-Alkoholen in Kosmetika ist an sich bekannt. Solche Species zeichnen sich dann meistens durch die Struktur 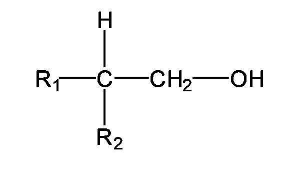 aus. Dabei bedeuten R₁ und R₂ in der Regel unverzweigte Alkylreste.

Erfindungsgemäß vorteilhaft werden der oder die Guerbet-Alkohole gewählt aus der Gruppe, bei denen
R₁ = Propyl, Butyl, Pentyl, Hexyl, Heptyl oder Octyl und
R₂ = Hexyl, Heptyl, Octyl, Nonyl, Decyl, Undecyl, Dodecyl, Tridecyl oder Tetradecyl.

Erfindungsgemäß bevorzugte Guerbet-Alkohole sind das 2-Butyloctanol - es hat die chemische Struktur 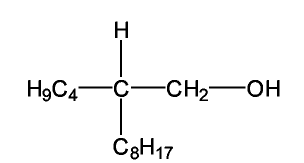 und ist beispielsweise unter der Handelsbezeichnung Isofol® 12 von der Gesellschaft Condea Chemie GmbH erhältlich - und das 2-Hexyldecanol - es hat die chemische Struktur 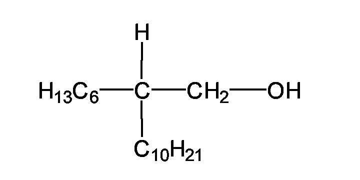 und ist beispielsweise unter der Handelsbezeichnung Isofol® 16 von der Gesellschaft Condea Chemie GmbH erhältlich. Auch Mischungen von erfindungsgemäßen Guerbet-Alkoholen sind erfindungsgemäß vorteilhaft zu verwenden. Mischungen aus 2-Butyloctanol und 2-Hexyldecanol sind beispielsweise unter der Handelsbezeichnung Isofol® 14 von der Gesellschaft Condea Chemie GmbH erhältlich.

Die Gesamtmenge an Guerbet-Alkoholen in den fertigen kosmetischen oder dermatologischen Zubereitungen wird vorteilhaft aus dem Bereich bis 25,0 Gew.-%, bevorzugt 0,5 - 15,0 Gew.-% gewählt, bezogen auf das Gesamtgewicht der Zubereitungen.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Besonders vorteilhafte mittelpolare Lipide im Sinne der vorliegenden Erfindung sind die im folgenden aufgelisteten Substanzen:

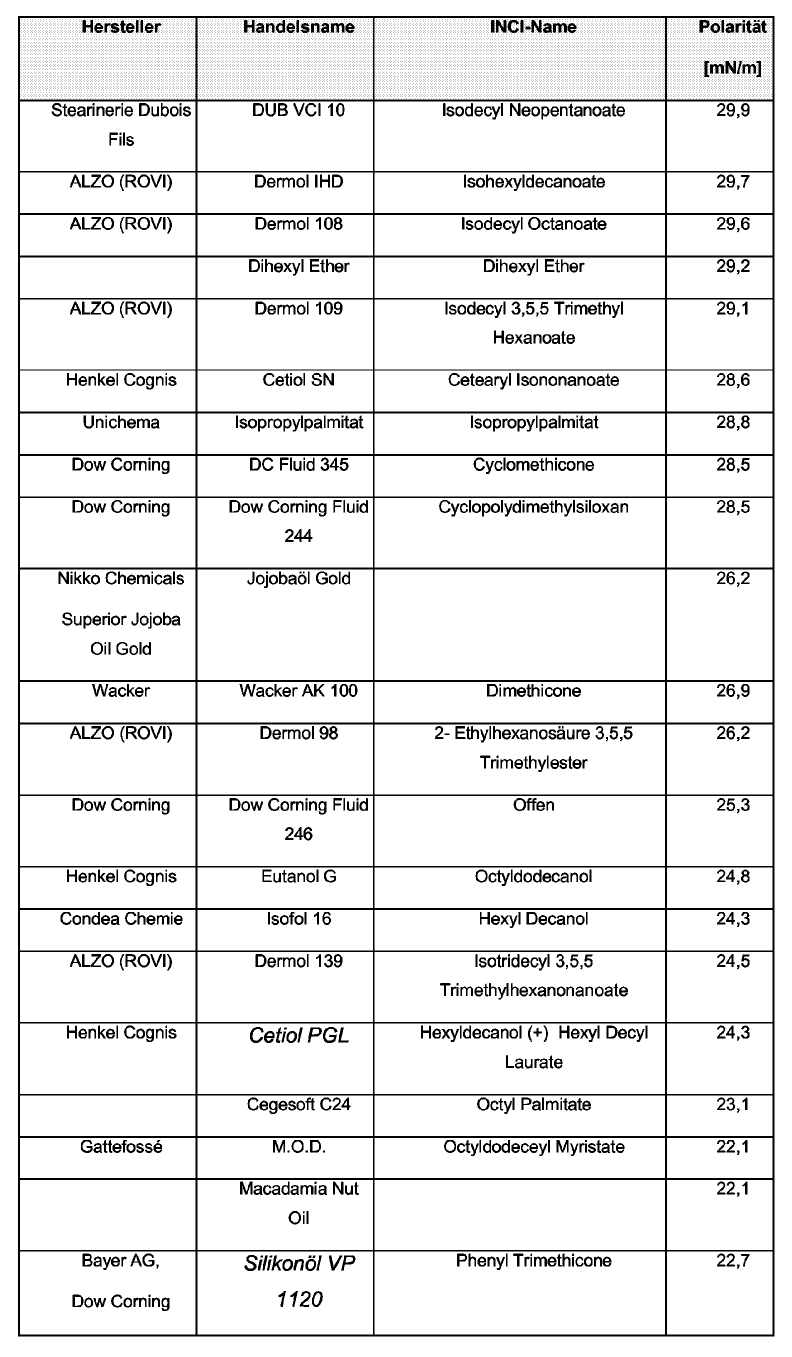

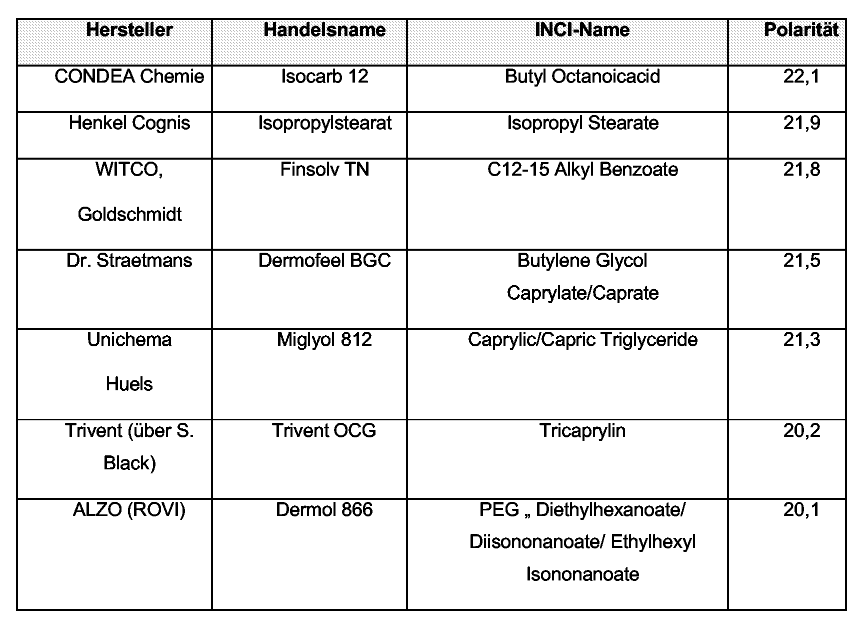

Unpolare Öle sind beispielsweise solche, welche gewählt werden aus der Gruppe der verzweigten und unverzweigten Kohlenwasserstoffe und -wachse, insbesondere Vaseline (Petrolatum), Paraffinöl, Squalan und Squalen, Polyolefine und hydrogenierte Polyisobutene. Unter den Polyolefinen sind Polydecene die bevorzugten Substanzen.

Besonders vorteilhafte unpolare Lipide im Sinne der vorliegenden Erfindung sind die im folgenden aufgelisteten Substanzen:

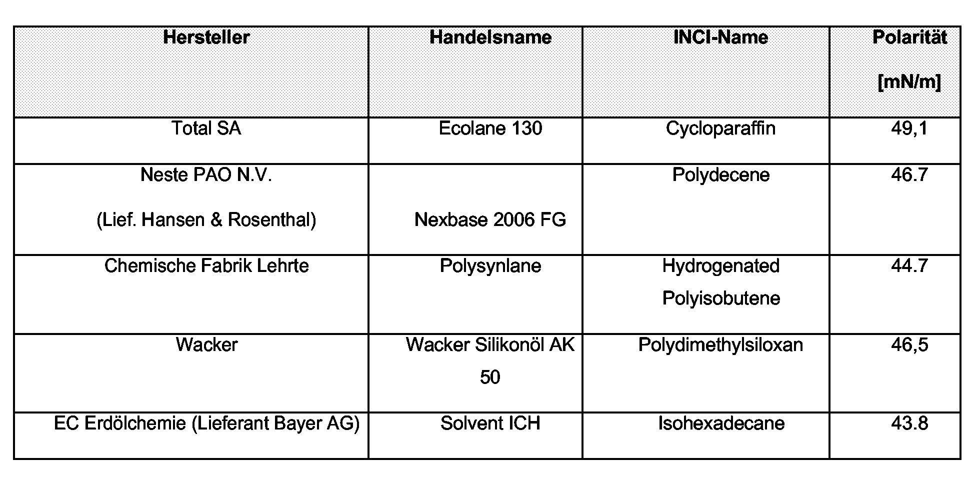

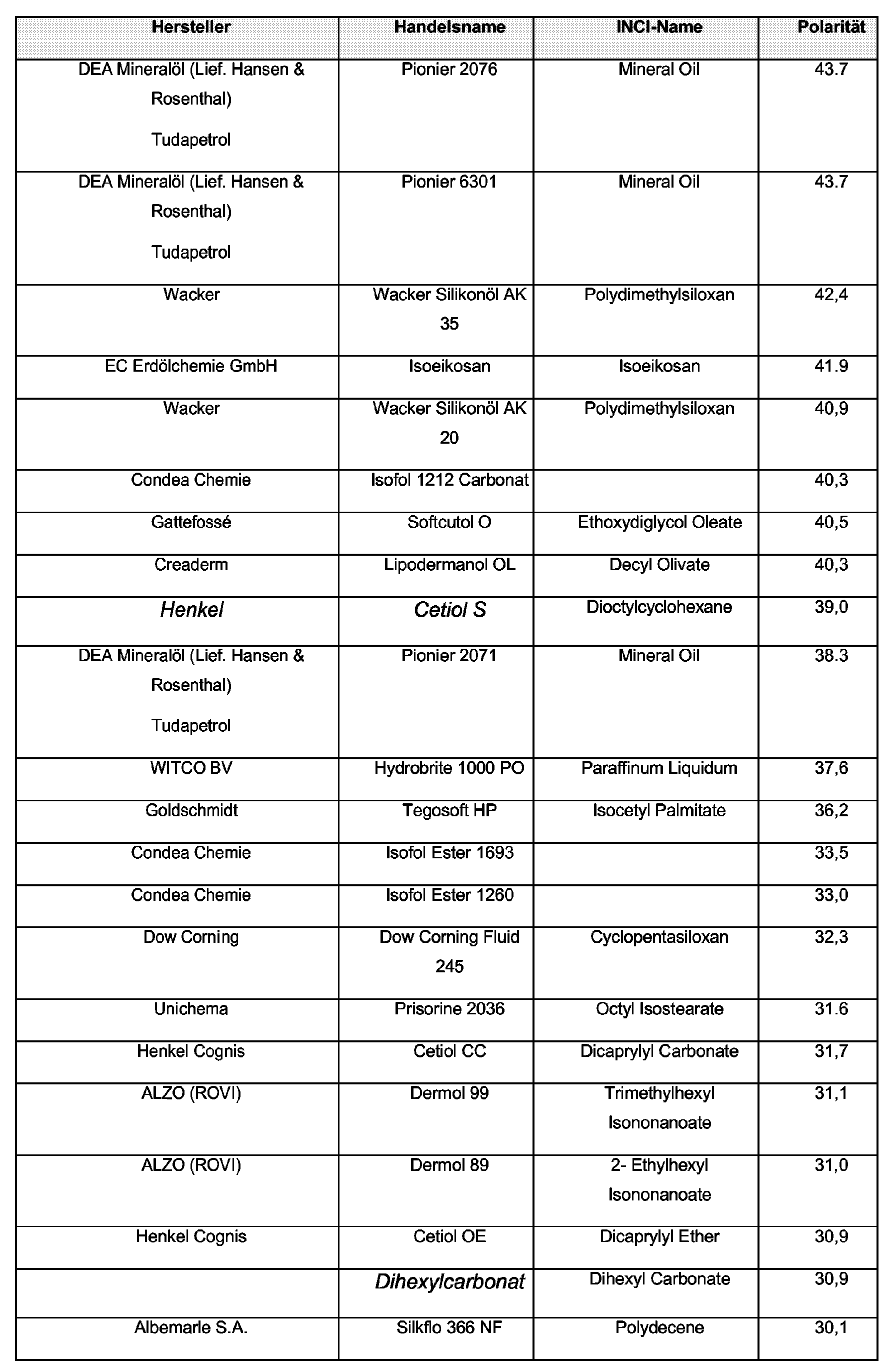

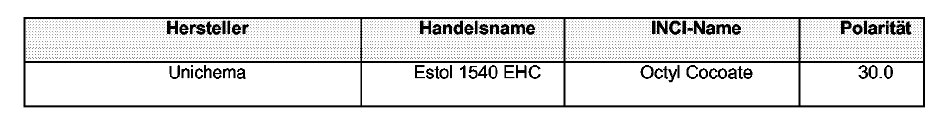

Es ist jedoch auch vorteilhaft, Gemische aus höher- und niederpolaren Lipiden und dergleichen zu verwenden. So kann die Ölphase vorteilhaft gewählt werden aus der Gruppe der verzweigten und unverzweigten Kohlenwasserstoffe und -wachse, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnußöl, Rapsöl, Mandelöl, Palmöl, Kokosöl, Palmkernöl und dergleichen mehr, sofern die im Hauptanspruch geforderten Bedingungen eingehalten werden.

Erfindungsgemäß vorteilhaft zu verwendende Fett- und/oder Wachskomponenten können aus der Gruppe der pflanzlichen Wachse, tierischen Wachse, Mineralwachse und petrochemischen Wachse gewählt werden. Erfindungsgemäß günstig sind beispielsweise Candelillawachs, Carnaubawachs, Japanwachs, Espartograswachs, Korkwachs, Guarumawachs, Reiskeimölwachs, Zuckerrohrwachs, Beerenwachs, Ouricurywachs, Montan-wachs, Jojobawachs, Shea Butter, Bienenwachs, Schellackwachs, Walrat, Lanolin (Wollwachs), Bürzelfett, Ceresin, Ozokerit (Erdwachs), Paraffinwachse und Mikrowachse, sofern die im Hauptanspruch geforderten Bedingungen eingehalten werden.

Weitere vorteilhafte Fett- und/oder Wachskomponenten sind chemisch modifzierte Wachse und synthetische Wachse, wie beispielsweise die unter den Handelsbezeichnungen Syncrowax HRC (Glyceryltribehenat), und Syncrowax AW 1C (C₁₈₋₃₆ -Fettsäure) bei der CRODA GmbH erhältlichen sowie Montanesterwachse, Sasolwachse, hydrierte Jojobawachse, synthetische oder modifizierte Bienenwachse (z. B. Dimethicon Copolyol Bienenwachs und/oder C₃₀₋₅₀ -Alkyl Bienenwachs), Polyalkylenwachse, Polyethylenglykolwachse, aber auch chemisch modifzierte Fette, wie z. B. hydrierte Pflanzenöle (beispielsweise hydriertes Ricinusöl und/oder hydrierte Cocosfettglyceride), Triglyceride, wie beispielsweise Trihydroxystearin, Fettsäuren, Fettsäureester und Glykolester, wie beispielsweise C₂₀₋₄₀-Alkylstearat, C₂₀₋₄₀-Alkylhydroxystearoylstearat und/oder Glykolmontanat. Weiter vorteilhaft sind auch bestimmte Organosiliciumverbindungen, die ähnliche physikalische Eigenschaften aufweisen wie die genannten Fett- und/oder Wachskomponenten, wie beispielsweise Stearoxytrimethylsilan sofern die im Hauptanspruch geforderten Bedingungen eingehalten werden.

Erfindungsgemäß können die Fett- und/oder Wachskomponenten sowohl einzeln als auch im Gemisch vorliegen. Auch beliebige Abmischungen solcher Öl- und Wachskomponenten sind vorteilhaft im Sinne der vorliegenden Erfindung einzusetzen.

Vorteilhaft wird die Ölphase gewählt aus der Gruppe 2-Ethylhexylisostearat, Octyldodecanol, Isotridecylisononanoat, Butylen Glycol Dicaprylat/Dicaprat, 2-Ethylhexylcocoat, C₁₂₋₁₅-Alkylbenzoat, Capryl-Caprinsäure-triglycerid, Dicaprylylether sofern die im Hauptanspruch geforderten Bedingungen eingehalten werden.

Besonders vorteilhaft sind Mischungen aus Octyldodecanol, Capryl-Caprinsäure-triglycerid, Dicaprylylether, Dicaprylyl Carbonat, Cocoglyceriden, oder Mischungen aus C₁₂₋₁₅-Alkybenzoat und 2-Ethylhexylisostearat, Mischungen aus C₁₂₋₁₅-Alkybenzoat und Butylen Glycol Dicaprylat/Dicaprat sowie Mischungen aus C₁₂₋₁₅-Alkybenzoat, 2-Ethylhexylisostearat und Isotridecylisononanoat sofern die im Hauptanspruch geforderten Bedingungen eingehalten werden.

Von den Kohlenwasserstoffen sind Paraffinöl, Cycloparaffin, Squalan, Squalen, hydriertes Polyisobuten bzw. Polydecen vorteilhaft im Sinne der vorliegenden Erfindung zu verwenden. sofern die im Hauptanspruch geforderten Bedingungen eingehalten werden.

In den Zubereitungen, in denen die erfindungsgemäße Verwendung der hydrierten Sojaglyceride verwirklicht ist, können ein oder mehrere Wachse in einer Gesamtkonzentration bis zu 10 Gewichts-%, bezogen auf das Gesamtgewicht der Zubereitung enthalten sein. Ferner können die Wachse einen Schmelzpunkt von größer als 30 °C und, in der Regel sogar Schmelzpunkte von 38- 55°C aufweisen.
Die erfindungsgemäßen Vorteile gegenüber dem Stande der Technik werden dabei insbesondere bei Wachsen mit einem Schmelzpunkt von 38-44 °C offenbar.

### Silikone

Es kann ebenfalls vorteilhaft sein, die Ölphase der erfindungsgemäßen Zubereitungen teilweise oder vollständig aus der Gruppe der cyclischen und/oder linearen Silicone zu wählen, welche im Rahmen der vorliegenden Offenbarung auch als "Siliconöle" bezeichnet werden. Solche Silicone oder Siliconöle können als Monomere vorliegen, welche in der Regel durch Strukturelemente charakterisiert sind, wie folgt:

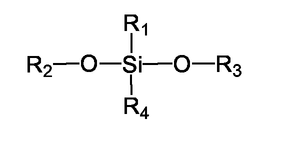

Silikonöle sind hochmolekulare synthetische polymere Verbindungen, in denen Silicium-Atome über Sauerstoff-Atome ketten- und/oder netzartig verknüpft und die restlichen Valenzen des Siliciums durch Kohlenwasserstoff-Reste (meist Methyl-, seltener Ethyl-, Propyl-, Phenyl-Gruppen u. a.) abgesättigt sind.

Als erfindungsgemäß vorteilhaft einzusetzenden linearen Silicone mit mehreren Siloxyleinheiten werden im allgemeinen durch Strukturelemente charakterisiert wie folgt: 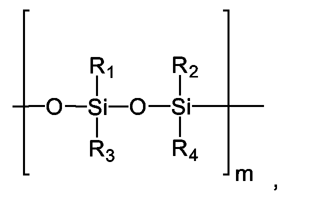 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). m kann dabei Werte von 2 - 200.000 annehmen.

Systematisch werden die linearen Silikonöle als Polyorganosiloxane bezeichnet; die methylsubstituierten Polyorganosiloxane, welche die mengenmäßig bedeutendsten Verbindungen dieser Gruppe darstellen und sich durch die folgende Strukturformel auszeichnen 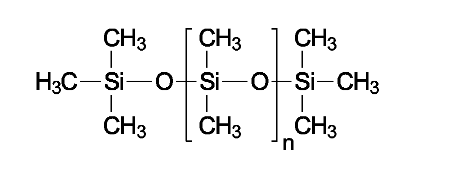 werden auch als Polydimethylsiloxan bzw. Dimethicon (INCI) bezeichnet. Dimethicone gibt es in verschiedenen Kettenlängen bzw. mit verschiedenen Molekulargewichten. Dimethicone unterschiedlicher Kettenlänge und Phenyltrimethicone sind besonders vorteilhafte lineare Silikonöle im Sinne der vorliegenden Erfindung.

Besonders vorteilhafte Polyorganosiloxane im Sinne der vorliegenden Erfindung sind ferner beispielsweise Dimethylpolysiloxane [Poly(dimethylsiloxan)], welche z. B. unter den Handelsbezeichnungen ABIL 10 bis 10 000 bei Th. Goldschmidt erhältlich sind. Ferner vorteilhaft sind Phenylmethylpolysiloxane (INCI: Phenyl Dimethicone, Phenyl Trimethicone), cyclische Silicone (Octamethylcyclotetrasiloxan bzw. Decamethylcyclopentasiloxan), welche nach INCI auch als Cyclomethicone bezeichnet werden, aminomodifizierte Silicone (INCI: Amodimethicone) und Siliconwachse, z. B. Polysiloxan-Polyalkylen-Copolymere (INCI: Stearyl Dimethicone und Cetyl Dimethicone) und Dialkoxydimethylpolysiloxane (Stearoxy Dimethicone und Behenoxy Stearyl Dimethicone), welche als verschiedene Abil-Wax-Typen bei Th. Goldschmidt erhältlich sind.

Besonders vorteilhaft im Sinne der vorliegenden Erfindung sind ferner die im folgenden aufgelisteten Silikonöle:

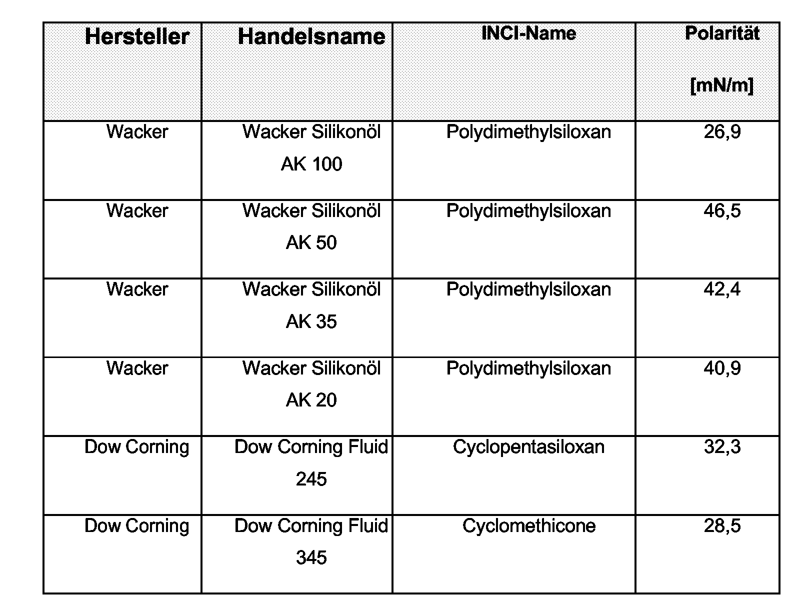

Erfindungsgemäß vorteilhaft einzusetzende cyclische Silicone werden im allgemeinen durch Strukturelemente charakterisiert, wie folgt 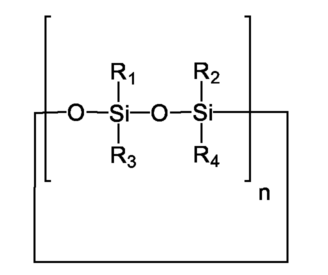 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). n kann dabei Werte von 3/2 bis 20 annehmen. Gebrochene Werte für n berücksichtigen, dass ungeradzahlige Anzahlen von Siloxylgruppen im Cyclus vorhanden sein können.

Besonders vorteilhafte cyclische Silikonöle im Sinne der vorliegenden Erfindung sind Cyclomethicone, insbesondere Cyclomethicone D5 und/oder Cyclomethicone D6.

Vorteilhafte Silkonöle bzw. Silikonwachse im Sinne der vorliegenden Erfindung sind cyclische und/oder lineare Silikonöle und Silikonwachse.

Es ist besonders vorteilhaft im Sinne der vorliegenden Erfindung das Verhältnis von Lipiden zu Silikonölen in etwa wie 1 : 1 (allgemein x : y) zu wählen.

Vorteilhaft wird Phenyltrimethicon als Siliconöl gewählt. Auch andere Silikonöle, beispielsweise Dimethicon, Phenyldimethicon, Cyclomethicon (Octamethylcyclotetrasiloxan) beispielsweise Hexamethylcyclotrisiloxan, Polydimethylsiloxan, Poly(methylphenylsiloxan), Cetyldimethicon, Behenoxydimethicon sind vorteilhaft im Sinne der vorliegenden Erfindung zu verwenden.

Vorteilhaft sind ferner Mischungen aus Cyclomethicon und Isotridecylisononanoat, sowie solche aus Cyclomethicon und 2-Ethylhexylisostearat.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol sowie das Cetyl-Dimethicon-Copolyol (und) Polyglyceryl-4-Isostearat (und) Hexyllaurat.

Die erfindungsgemäßen Zubereitungen können darüber hinaus weitere kosmetische und/oder dermatologische Wirk-, Hilfs- und Zusatzstoffe enthalten.

Erfindungsgemäß vorteilhafte Kosmetika sind beispielsweise dadurch gekennzeichnet, dass ein oder mehrere Wirkstoffe gewählt werden aus der Gruppe der UV-Lichtschutzfilter, Vitamine, Vitaminderivate, Antioxidantien, Antifaltenwirkstoffe, Selbstbräuner, Depigmentiermittel, Repellentien, Pflanzenextrakte, Enzyme, wasserfreie Polyole, Farbstoffe, Effektstoffe.

Als erfindungsgemäß vorteilhafte UV-Lichtschutzfilter können beispielsweise die folgenden Verbindungen eingesetzt werden:

### Anorganische Pigmente

Bevorzugte anorganische Pigmente sind Metalloxide und/oder andere in Wasser schwerlösliche oder unlösliche Metallverbindungen, insbesondere Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxide der entsprechenden Metalle sowie Abmischungen aus solchen Oxiden, sowie das Sulfat des Bariums (BaSO₄).

Die Titandioxid- Pigmente können sowohl in der Kristallmodifikation Rutil als auch Anatas vorliegen und können im Sinne der vorliegenden Erfindung vorteilhaft oberflächlich behandelt ("gecoatet") sein, wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtung können im Sinne der vorliegenden Erfindung auch Wasser enthalten.

Beschriebene beschichtete und unbeschichtete Titandioxide können im Sinne vorliegender Erfindung auch in Form kommerziell erhältlicher öliger oder wässriger Vordispersionen zur Anwendung kommen. Diesen Vordispersionen können vorteilhaft Dispergierhilfmittel und/oder Solubilisationsvermittler zugesetzt sein.

Die erfindungsgemäßen Titandioxide zeichnen sich durch eine Primärpartikelgröße zwischen 10 nm bis 150 nm aus.

| **Handelsname** | **Coating** | **zusätzliche Bestandteile der Vordispersion** | **Hersteller** |
|---|---|---|---|
| MT-100TV | Aluminiumhydroxid Stearinsäure | - | Tayca Corporation |
| MT-100Z | Aluminiumhydroxid Stearinsäure | - | Tayca Corporation |
| MT-100F | Stearinsäure Eisenoxid | - | Tayca Corporation |
| MT-500SAS | Alumina, Silica Silikon | - | Tayca Corporation |
| MT-100AQ | Silica Aluminiumhydroxid Alginsäure | - | Tayca Corporation |
| Eusolex T-2000 | Alumina Simethicone | - | Merck KgaA |
| Eosolex TS | Alumina, Stearinsäure | - | Merck KgaA |
| Titandioxid P25 | None | - | Degussa |
| Titandioxid T805 (Uvinul TiO₂) | Octyltrimethylsilan | - | Degussa |
| UV-Titan X170 | Alumina Dimethicone | - | Kemira |
| UV-Titan X161 | Alumina, Silica Stearinsäure | - | Kemira |
| Tioveil AQ 10PG | Alumina Silica | Wasser Propylenglycol | Solaveil Uniquema |
| Mirasun TiW 60 | Alumina Silica | Wasser | Rhone-Poulenc |

Im Sinne der vorliegenden Erfindung sind besonders bevorzugte Titandioxide das MT-100 Z und MT-100 TV von Tayca Corporation, Eusolex T-2000 und Eusolex TS von Merck und das Titandioxid T 805 von Degussa.

Zinkoxide können im Sinne der vorliegenden Erfindung auch in Form kommerziell erhältlicher öliger oder wässriger Vordispersionen zur Anwendung kommen. Erfindungsgemäß geeignete Zinkoxidpartikel und Vordispersionen von Zinkoxidpartikeln zeichnen sich durch eine Primärpartikelgröße von < 300 nm aus und sind unter folgenden Handelsbezeichnungen bei den aufgeführten Firmen erhältlich:

| **Handelsname** | **Coating** | **Hersteller** |
|---|---|---|
| Z- Cote HP1 | 2% Dimethicone | BASF |
| Z- Cote | / | BASF |
| ZnO NDM | 5% Dimethicone | H&R |
| MZ 707M | 7% Dimethicone | M. Tayca Corp. |
| Nanox 500 | / | Elementis |
| ZnO Neutral | / | H&R |

Besonderes bevorzugte Zinkoxide im Sinne der Erfindung sind das Z-Cote HP1 von der Firma BASF und das Zinkoxid NDM von der Firma Haarmann & Reimer.

Die Gesamtmenge an einem oder mehreren anorganischen Pigmenten in der fertigen kosmetischen Zubereitung wird vorteilhaft aus dem Bereich 0,1 Gew.-% bis 25 Gew.-% gewählt, vorzugsweise 0,5 Gew.-% bis 18 Gew.-%.

### Organische Pigmente

Vorteilhaftes organisches Pigment im Sinne der vorliegenden Erfindung ist das 2,2'-Methylen-bis-(6-(2H-benzotriazol-2-yl)-4-(1,1,3,3-tetramethylbutyl)-phenol) [INCI: Bisoctyltriazol], welches unter der Handelsbezeichnung Tinosorb® M bei der CIBA-Chemikalien GmbH erhältlich ist.

### Weitere UV-Lichtschutzfilter

Vorteilhafte UV-A-Filtersubstanzen im Sinne der vorliegenden Erfindung sind Dibenzoylmethanderivate, insbesondere das 4-(tert.-Butyl)-4'-methoxydibenzoylmethan (CAS-Nr. 70356-09-1), welches von Givaudan unter der Marke Parsol® 1789 und von Merck unter der Handelsbezeichnung Eusolex® 9020 verkauft wird.

Weitere vorteilhafte UV-A-Filtersubstanzen sind die Phenylen-1,4-bis-(2-benzimidazyl)-3,3'-5,5'-tetrasulfonsäure und ihre Salze, besonders die entsprechenden Natrium-, Kalium- oder Triethanolammonium-Salze, insbesondere das Phenylen-1,4-bis-(2-benzimidazyl)-3,3'-5,5'-tetrasulfonsäure-bis-natriumsalz mit der INCI-Bezeichnung Bisimidazylate, welches beispielsweise unter der Handelsbezeichnung Neo Heliopan AP bei Haarmann & Reimer erhältlich ist.

Ferner vorteilhaft sind das 1,4-di(2-oxo-10-Sulfo-3-bornylidenmethyl)-Benzol und dessen Salze (besonders die entprechenden 10-Sulfato-verbindungen, insbesondere das entsprechende Natrium-, Kalium- oder Triethanolammonium-Salz), das auch als Benzol-1,4-di(2-oxo-3-bornylidenmethyl-10-sulfonsäure) bezeichnet wird.

Weitere vorteilhafte UV-A-Filtersubstanzen sind Hydroxybenzophenone, die sich durch die folgende Strukturformel auszeichnen: 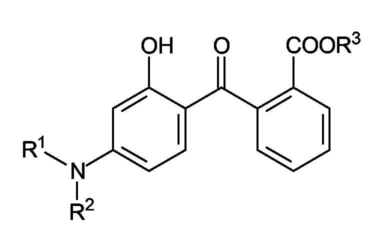 worin
- R¹ und R² unabhängig voneinander Wasserstoff, C₁-C₂₀-Alkyl, C₃-C₁₀-Cycloalkyl oder C₃-C₁₀-Cycloalkenyl bedeuten, wobei die Substituenten R¹ und R² gemeinsam mit dem Stickstoffatom, an das sie gebunden sind, einen 5- oder 6-Ring bilden können und
- R³ einen C₁-C₂₀-Alkyl Rest bedeutet.

Ein besonders vorteilhaftes Hydroxybenzophenon im Sinne der vorliegenden Erfindung ist der 2-(4'-Diethylamino-2'-hydoxybenzoyl)-benzoesäurehexylester (auch: Aminobenzophenon), welcher unter dem Handelsnamen Uvinul A Plus bei der Fa. BASF erhältlich ist.

Vorteilhafte UV-Filtersubstanzen im Sinne der vorliegenden Erfindung sind ferner sogenannte Breitbandfilter, d.h. Filtersubstanzen, die sowohl UV-A- als auch UV-B-Strahlung absorbieren.

Vorteilhafte Breitbandfilter oder UV-B-Filtersubstanzen sind beispielsweise Bis-Resorcinyltriazinderivate mit der folgenden Struktur: 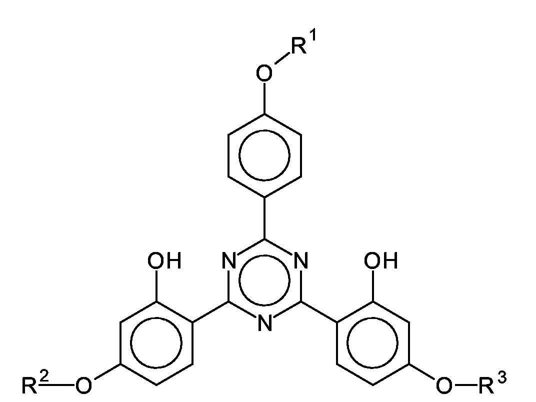 wobei R¹, R² und R³ unabhängig voneinander gewählt werden aus der Gruppe der verzweigten und unverzweigten Alkylgruppen mit 1 bis 10 Kohlenstoffatomen bzw. ein einzelnes Wasserstoffatom darstellen. Insbesondere bevorzugt sind das 2,4-Bis-{[4-(2-Ethyl-hexyloxy)-2-hydroxy]-phenyl}-6-(4-methoxyphenyl)-1,3,5-triazin (INCI: Aniso Triazin), welches unter der Handelsbezeichnung Tinosorb® S bei der CIBA-Chemikalien GmbH erhältlich ist.

Besonders vorteilhafte Zubereitungen im Sinne der vorliegenden Erfindung, die sich durch einen hohen bzw. sehr hohen UV-A-Schutz auszeichnen, enthalten bevorzugt mehrere UV-A- und/oder Breitbandfilter, insbesondere Dibenzoylmethanderivate [beispielsweise das 4-(tert.-Butyl)-4'-methoxydibenzoylmethan], Benzotriazolderivate [beispielsweise das 2,2'-Methylen-bis-(6-(2H-benzotriazol-2-yl)-4-(1,1,3,3-tetramethylbutyl)-phenol)], Phenylen-1,4-bis-(2-benzimidazyl)-3,3'-5,5'-tetrasulfonsäure und/oder ihre Salze, das 1,4-di(2-oxo-10-Sulfo-3-bornylidenmethyl)-Benzol und/oder dessen Salze und/oder das 2,4-Bis-([4-(2-Ethyl-hexyloxy)-2-hydroxy]-phenyl}-6-(4-methoxyphenyl)-1,3,5-triazin, jeweils einzeln oder in beliebigen Kombinationen miteinander.

Auch andere UV-Filtersubstanzen, welche das Strukturmotiv 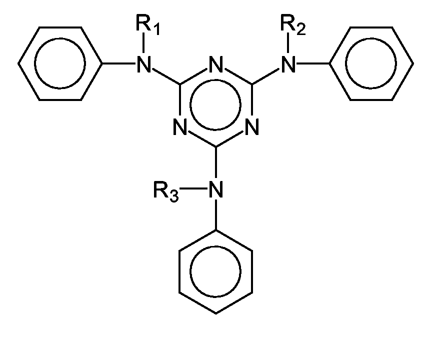 aufweisen, sind vorteilhafte UV-Filtersubstanzen im Sinne der vorliegenden Erfindung, beispielsweise die in der Europäischen Offenlegungsschrift EP 570 838 A1 beschriebenen s-Triazinderivate, deren chemische Struktur durch die generische Formel 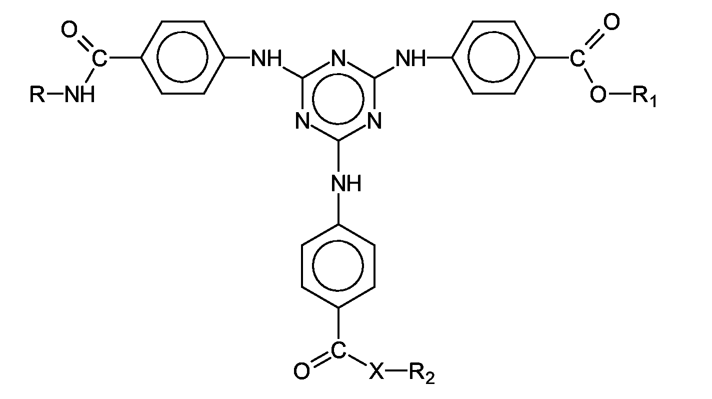 wiedergegeben wird, wobei
- R: einen verzweigten oder unverzweigten C₁-C₁₈-Alkylrest, einen C₅-C₁₂-Cycloalkylrest, gegebenenfalls substituiert mit einer oder mehreren C₁-C₄- Alkylgruppen, darstellt,
- X: ein Sauerstoffatom oder eine NH-Gruppe darstellt,
- R₁: einen verzweigten oder unverzweigten C₁-C₁₈-Alkylrest, einen C₅-C₁₂-Cycloalkylrest, gegebenenfalls substituiert mit einer oder mehreren C₁-C₄- Al kylgruppen, oder ein Wasserstoffatom, ein Alkalimetallatom, eine Ammoniumgruppe oder eine Gruppe der Formel 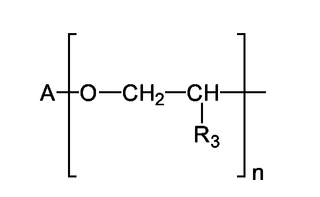 bedeutet, in welcher
- A: einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
- R₃: ein Wasserstoffatom oder eine Methylgruppe darstellt,
- n: eine Zahl von 1 bis 10 darstellt,
- R₂: einen verzweigten oder unverzweigten C₁-C₁₈-Alkylrest, einen C₅-C₁₂-Cycloalkylrest, gegebenenfalls substituiert mit einer oder mehreren C₁-C₄- AI kylgruppen, darstellt, wenn X die NH-Gruppe darstellt, und
einen verzweigten oder unverzweigten C₁-C₁₈-Alkylrest, einen C₅-C₁₂-Cycloalkylrest, gegebenenfalls substituiert mit einer oder mehreren C₁-C₄- AI kylgruppen, oder ein Wasserstoffatom, ein Alkalimetallatom, eine Ammoniumgruppe oder eine Gruppe der Formel 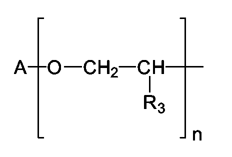 bedeutet, in welcher
A einen verzweigten oder unverzweigten C₁-C₁₈-Alkylrest, einen C₅-C₁₂-Cycloalkyl- oder Arylrest darstellt, gegebenenfalls substituiert mit einer oder mehreren C₁-C₄- Alkylgruppen,
R₃ ein Wasserstoffatom oder eine Methylgruppe darstellt,
n eine Zahl von 1 bis 10 darstellt,
wenn X ein Sauerstoffatom darstellt.

Besonders bevorzugte UV-Filtersubstanz im Sinne der vorliegenden Erfindung ist ferner ein unsymmetrisch substituiertes s-Triazin, dessen chemische Struktur durch die Formel 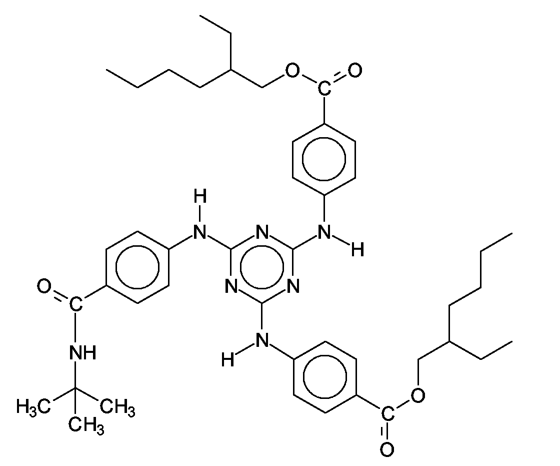 wiedergegeben wird, welches im Folgenden auch als Dioctylbutylamidotriazon (INCI: Dioctylbutamidotriazone) bezeichnet wird und unter der Handelsbezeichnung UVASORB HEB bei Sigma 3V erhältlich ist.

Vorteilhaft im Sinne der vorliegenden Erfindung ist auch ein symmetrisch substituiertes s-Triazin, das 4,4',4"-(1,3,5-Triazin-2,4,6-triyltriimino)-tris-benzoesäure-tris(2-ethylhexylester), synonym: 2,4,6-Tris-[anilino-(p-carbo-2'-ethyl-1'-hexyloxy)]-1,3,5-triazin (INCI: Octyl Triazone), welches von der BASF Aktiengesellschaft unter der Warenbezeichnung UVINUL® T 150 vertrieben wird.

Auch in der Europäischen Offenlegungsschrift 775 698 werden bevorzugt einzusetzende Bis-Resorcinyltriazinderivate beschrieben, deren chemische Struktur durch die generische Formel 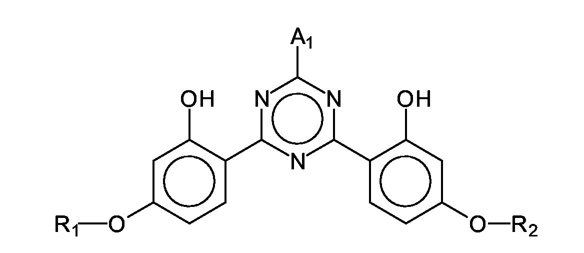 wiedergegeben wird, wobei R₁ , R₂ und A₁ verschiedenste organische Reste repräsentieren.

Vorteilhaft im Sinne der vorliegenden Erfindung sind ferner das 2,4-Bis-{[4-(3-sulfonato)-2-hydroxy-propyloxy)-2-hydroxy]-phenyl}-6-(4-methoxyphenyl)-1,3,5-triazin Natriumsalz, das 2,4-Bis-{[4-(3-(2-Propyloxy)-2-hydroxy-propyloxy)-2-hydroxy]-phenyl}-6-(4-methoxyphenyl)-1,3,5-triazin, das 2,4-Bis-{[4-(2-ethyl-hexyloxy)-2-hydroxy]-phenyl}-6-[4-(2-methoxyethylcarboxyl)-phenylamino]-1,3,5-triazin, das 2,4-Bis-{[4-(3-(2-propyloxy)-2-hydroxy-propyloxy)-2-hydroxy]-phenyl}-6-[4-(2-ethyl-carboxyl)-phenylamino ]-1,3,5-triazin, das 2,4-Bis-{[4-(2-ethyl-hexyloxy)-2-hydroxy]-phenyl)-6-(1-methyl-pyrrol-2-yl)-1,3,5-triazin, das 2,4-Bis-{[4-tris(trimethylsiloxy-silylpropyloxy)-2-hydroxy]-phenyl}-6-(4-methoxyphenyl)-1,3,5-triazin, das 2,4-Bis-{[4-(2"-methylpropenyloxy)-2-hydroxy]-phenyl}-6-(4-methoxyphenyl)-1,3,5-triazin und das 2,4-Bis-{[4-(1',1',1',3',5',5',5'-Heptamethylsiloxy-2"-methyl-propyloxy)-2-hydroxy]-phenyl}-6-(4-methoxyphenyl)-1,3,5-triazin.
Ein vorteilhafter Breitbandfilter im Sinne der vorliegenden Erfindung ist das 2,2'-Methylen-bis-(6-(2H-benzotriazol-2-yl)-4-(1,1,3,3-tetramethylbutyl)-phenol), welches unter der Handelsbezeichnung Tinosorb® M bei der CIBA-Chemikalien GmbH erhältlich ist.

Vorteilhafter Breitbandfilter im Sinne der vorliegenden Erfindung ist ferner das 2-(2H-benzotriazol-2-yl)-4-methyl-6-[2-methyl-3-[1,3,3,3-tetramethyl-1-[(trimethylsilyl)oxy]disiloxanyl]propyl]-phenol (CAS-Nr.: 155633-54-8) mit der INCI-Bezeichnung Drometrizole Trisiloxane.

Die UV-B- und/oder Breitband-Filter können öllöslich oder wasserlöslich sein. Vorteilhafte öllösliche UV-B- und/oder Breitband-Filtersubstanzen sind z. B.:
■ 3-Benzylidencampher-Derivate, vorzugsweise 3-(4-Methylbenzyliden)campher, 3-Benzylidencampher;
■ 4-Aminobenzoesäure-Derivate, vorzugsweise 4-(Dimethylamino)-benzoesäure(2-ethylhexyl)ester, 4-(Dimethylamino)benzoesäureamylester;
■ 2,4,6-Trianilino-(p-carbo-2'-ethyl-1'-hexyloxy)-1,3,5-triazin;
■ Ester der Benzalmalonsäure, vorzugsweise 4-Methoxybenzalmalonsäuredi(2-ethylhexyl)-ester;
■ Ester der Zimtsäure, vorzugsweise 4-Methoxyzimtsäure(2-ethylhexyl)ester, 4-Methoxyzimtsäureisopentylester;
■ Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon
■ sowie an Polymere gebundene UV-Filter.

Vorteilhafte wasserlösliche UV-B- und/oder Breitband-Filtersubstanzen sind z. B.:
■ Salze der 2-Phenylbenzimidazol-5-sulfonsäure, wie ihr Natrium-, Kalium- oder ihr Triethanolammonium-Salz, sowie die Sulfonsäure selbst;
■ Sulfonsäure-Derivate des 3-Benzylidencamphers, wie z. B. 4-(2-Oxo-3-bornylidenmethyl)benzolsulfonsäure, 2-Methyl-5-(2-oxo-3-bornylidenmethyl)sulfonsäure und deren Salze.

Besonders vorteilhafte bei Raumtemperatur flüssige UV-Filtersubstanzen im Sinne der vorliegenden Erfindung sind Homomenthylsalicylat (INCI: Homosalate), 2-Ethylhexyl-2-hydroxybenzoat (2-Ethylhexylsalicylat, Octylsalicylat, INCI: Octyl Salicylate), 4-Isopropylbenzylsalicylat und Ester der Zimtsäure, vorzugsweise 4-Methoxyzimtsäure(2-ethylhexyl)ester (2-Ethylhexyl-4-methoxycinnamat, INCI: Octyl Methoxycinnamate) und 4-Methoxyzimtsäureisopentylester (Isopentyl-4-methoxycinnamat, INCI: Isoamyl p-Methoxycinnamate), 3-(4-(2,2-bis Ethoxycarbonylvinyl)-phenoxy)propenyl)-methoxysiloxan/Dimethylsiloxan - Copolymer (INCI: Dimethicodiethylbenzalmalonat) welches beispielsweise unter der Handelsbezeichnung Parsol® SLX bei Hoffmann La Roche erhältlich ist.

Eine weiterere erfindungsgemäß vorteilhaft zu verwendende Lichtschutzfiltersubstanz ist das Ethylhexyl-2-cyano-3,3-diphenylacrylat (Octocrylen), welches von BASF unter der Bezeichnung Uvinul® N 539 erhältlich ist.

Es kann auch von erheblichem Vorteil sein, polymergebundene oder polymere UV-Filtersubstanzen in Zubereitungen gemäß der vorliegenden Erfindung zu verwenden, insbesondere solche, wie sie in der WO-A-92/20690 beschrieben werden.

Die Liste der genannten UV-Filter, die im Sinne der vorliegenden Erfindung eingesetzt werden können, soll selbstverständlich nicht limitierend sein.

Vorteilhaft enthalten die erfindungsgemäßen Zubereitungen die Substanzen, die UV-Strahlung im UV-A- und/oder UV-B-Bereich absorbieren, in einer Gesamtmenge von z. B. 0,1 Gew.-% bis 30 Gew.-%, vorzugsweise 0,5 bis 20 Gew.-%, insbesondere 1,0 bis 15,0 Gew.-%, jeweils bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar oder die Haut dienen.

### Vitamine und Vitaminderivate

Hierzu zählen unter anderem die Vitamine A, B₁₋₆, B₁₂, C, D, E, F, H, K und PP sowie deren Derivate. Diese können erfindungsgemäß vorteilhaft in einer Konzentration von 0,001 bis 10 Gew.-%, bevorzugt 0,05 - 7 Gew.-%, insbesondere bevorzugt 0,5 - 5 Gew.-%, bezogen auf das Gesamtgewicht der Zubereitung, in welcher sie enthalten sind, enthalten sein.

Als erfindungsgemäß bevorzugte Vitaminderivate werden dabei Retinylpalmitat, Ascorbylglucosid, Tocopherylacetat, Tocopherylpalmitat, Niacinamid, Panthenol eingesetzt.

Erfindungsgemäß vorteilhaft ist der Einsatz von Vitaminen und deren Derivaten in einer Konzentration von 0,05 bis 1 Gewichts- %, bevorzugt in einer Konzentration von 0,01 bis 0,5 Gewichts-% und ganz besonders bevorzugt in einer Konzentration von 0,05 bis 0,2 Gewichts-%, bezogen auf das Gesamtgewicht der Zubereitung.

### Antioxidantien

Vorteilhaft werden die Antioxidantien gewählt aus der Gruppe bestehend aus Aminosäuren (z.B. Glycin, Lysin, Arginin, Cystein, Histidin, Tyrosin, Tryptophan) und deren Derivate (als Salz-, Ester-, Ether-, Zucker-, Nukleotid-, Nukleosid-, Peptid- und Lipid-Verbidung), Imidazole (z.B. Urocaninsäure) und deren Derivate (als Salz-, Ester-, Ether-, Zucker-, Nukleotid-, Nukleosid-, Peptid- und/o der Lipid-Verbidung), Peptide wie D,L-Carnosin, D-Carnosin, L-Carnosin, Anserin und deren Derivate (z.B. als Salz-, Ester-, Ether-, Zucker-, Thiol-, Nukleotid-, Nukleosid-, Peptid- und Lipid-Verbidung), Carotinoide, Carotine (z.B. α-Carotin, β-Carotin, ψ-Lycopin, Phytoen, ) und deren Derivate (z. B. als Salz-, Ester-, Ether-, Zucker-, Nukleotid-, Nukleosid-, Peptid- und/oder Lipid-Verbidung), Chlorogensäure und deren Derivate (als Salz-, Ester-, Ether-, Zucker-, Thiol-, Nukleotid-, Nukleosid-, Peptid- und/oder Lipid-Verbidung), Aurothioglucose, Propylthiouracil und andere Thiole (z.B. Thioredoxin, Liponsäure, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (als Salz-, Ester-, Ether-, Zucker-, Thiol-, Nukleotid-, Nukleosid-, Peptid- und/oder Lipid-Verbidung) sowie Sulfoximinverbindungen (z.B. Homocysteinsulfoximin, Buthioninsulfone, Penta-, Hexa-, Heptathioninsulfoximin) in sehr geringen verträglichen Dosierungen (z.B. pmol bis µmol/kg). Ferner (Metall)-Chelatoren (z.B. Apoferritin, Desferral, Lactoferrin, α-Hydroxyfettsäuren, Palmitinsäure, Phytinsäure) und deren Derivate (als Salz-, Ester-, Ether-, Zucker-, Thiol-, Nukleotid-, Nukleosid-, Peptid- und/oder Lipid-Verbidung), α-Hydroxysäuren (z.B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Furfurylidensorbitol und dessen Derivate, Ubichinon, Ubichinol, Plastochinon und deren Derivate (als Salz-, Ester-, Ether-, Zucker-, Thiol-, Nukleotid-, Nukleosid-, Peptid- und Lipid-Verbidung), Vitamin C und Derivate (z.B. Ascorbylpalmitat, Mg-Ascorbylphosphat, Ascorbylacetat, Natriumascorbylphosphate, Tocophe role und Derivate (z.B. Vitamin-E-acetat), sowie Phenolische Verbindungen und Pflanzenextrakte, diese enthaltend, wie z. B. Flavonoide (z. B. Glycosylrutin, Ferulasäure, Kaffeesäure), Furfurylidenglucitol, Butylhydroxytoluol, Butylhydroxyanisol, Nordihydroguajakharzsäure, Nordihydroguajaretsäure, Trihydroxybutyrophenon und deren Derivate (als Salz-, Ester-, Ether-, Zucker-, Nukleotid-, Nukleosid-, Peptid- und Lipid-Verbidung). Harnsäure und deren Derivate, Mannose und deren Derivate (als Salz-, Ester-, Ether-, Zucker-, Thiol-, Nukleotid-, Nukleosid-, Peptid- und Lipid-Verbidung). Zink und dessen Derivate (z.B. ZnO, ZnSO₄), Selen und dessen Derivate (z.B. Selenmethionin, Ebselen), Stilbene und deren Derivate (z.B. Stilbenoxid, Trans-Stilbenoxid) und die erfindungsgemäß geeigneten Derivate (als Salz-, Ester-, Ether-, Zucker-, Thiol-, Nukleotid-, Nukleosid-, Peptid- und/oder Lipid-Verbidung) dieser genannten Wirkstoffe.

Die Menge der vorgenannten Antioxidantien (eine oder mehrere Verbindungen) in den Zubereitungen beträgt vorzugsweise 0,001 bis 30 Gew.-%, besonders bevorzugt 0,05 - 20 Gew.-%, insbesondere 1 - 10 Gew.-%, bezogen auf das Gesamtgewicht der Zubereitung.

Sofern Vitamin E und/oder dessen Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist vorteilhaft, deren jeweilige Konzentrationen aus dem Bereich von 0,001 - 10 Gew.-%, bezogen auf das Gesamtgewicht der Formulierung, zu wählen.

### Antifaltenwirkstoffe, Pflanzenextrakte und andere Wirkstoffe

Weitere vorteilhafte Wirkstoffe im Sinne der vorliegenden Erfindung sind natürliche Wirkstoffe und/oder deren Derivate, wie z. B. alpha-Liponsäure, Phytoen, D-Biotin, Coenzym Q10, alpha-Glucosylrutin, Carnitin, Carnosin, natürliche und/oder synthetische Isoflavonoide, Kreatin, Kreatinin, Lignane, Taurin und/oder β-Alanin.

Erfindungsgemäß vorteilhaft können aber auch andere pharmazeutisch oder dermatologisch wirkende Substanzen wie beispielsweise die Haut beruhigende und pflegende Substanzen eingearbeitet sein. Hierzu zählen beispielsweise Panthenol, Allantoin, Tannin sowie Pflanzenwirkstoffe wie Azulen und Bisabolol, Glycyrrhizin, Hamamelin und Pflanzenextrakte wie Kamille, aloe vera, Hamazelis, Süßholzwurzel.

Die Menge der vorgenannten Wirkstoffe (eine oder mehrere Verbindungen) in den Zubereitungen beträgt vorzugsweise 0,001 bis 30 Gew.-%, besonders bevorzugt 0,05 - 20 Gew.-%, insbesondere 1-10 Gew.-%, bezogen auf das Gesamtgewicht der Zubereitung.

### Selbstbräuner

Als Selbstbräuner werden erfindungsgemäß vorteilhaft unter anderem eingesetzt: Glycerolaldehyd, Hydroxymethylglyoxal, γ-Dialdehyd, Erythrulose, 6-Aldo-D-Fructose, Ninhydrin, 5-Hydroxy-1,4-naphtochinon (Juglon), 2-Hydroxy-1,4-naphtochinon (Lawson).

Besonders bevorzugt im Sinne der Erfindung ist das 1,3-Dihydroxyaceton (DHA). Von Vorteil ist dabei eine Konzentration von 0,5 bis 10 Gewichts-% 1,3-Dihydroxyaceton und besonders eine Konzentration von 1 bis 7 Gewichts-%, bezogen auf das Gesamtgewicht der Zubereitung.

### Depigmentiermittel

Als erfindungsgemäß vorteilhafte Depigmentierungsmittel können beispielsweise Dicarbonsäuren wie 8-Hexadecen-1,16-dicarbonsäure (Dioic acid, CAS-Nummer 20701-68-2), Kojisäure, Ascorbinsäure und Azelainsäure sowie deren Derivate eingesetzt werden.

Erfindungsgemäß vorteilhaft beträgt die Gesamtkonzentration an Depigmentierungsmitteln in der entsprechenden Zubereitung 0,001 - 10 Gew.-%, bevorzugt 0,005 - 8 Gew.-%, insbesondere 0,05 - 5 Gew.-%.

### Repellentien

Erfindungsgemäß vorteilhaft können eingesetzt werden:
N,N-Diethyl-3-methylbenzamid (DEET), Dimethylphthalat (Palatinol M, DMP), 2,3;4,5-bis-(2-Butylen)-tetrahydro-2-furaldehyd (MGK-Repellent 11), Butopyronoxyl (Indalone), N,N-Caprylsäurediethylamid (Repellent 790), o-Chlor-N,N-diethylbenzamid in Mischung mit N,N-Diethylbenzamid (Kik-Repellent), Dimethylcarbat (Dimalone), Di-n-propylisocinchomeronat (MGK-Repellent 326), 2-Ethylhexan-1,3-diol (Rutgers 612), N-Octyl-bicycloheptendicarboximid (MGK 264 Insecticidesynergist), Piperonyl-butoxid (PBO), 1-Piperidincarbonsäure-2-(2-hydroxyethyl)-1-methylpropylester (Bayrepel® ) und 3-(N-n-Butyl-N-acetyl-amino)propionsäureethylester (auch als Repellent 3535 bezeichnet).

Erfindungsgemäß vorteilhaft ist ein Gehalt an Repellentien von 0,005 bis 70,0 Gew.-% eingesetzt, insbesondere 0,01 bis 50,0 Gew.-% und ganz besonders bevorzugt 3 bis 15 Gew.-% bezogen auf das Gesamtgewicht der Zubereitung.

### Farb-und Effektstoffe

Als erfindungsgemäß vorteilhafte pigmentäre Farbstoffe können alle aus der entsprechenden Positivliste der Kosmetikverordnung bzw. der EG-Liste kosmetischer Färbemittel aufgelisteteten Verbindungen eingesetzt werden. In den meisten Fällen sind sie mit den für Lebensmittel zugelassenen Farbstoffen identisch. Neben Farbpigmenten können die erfindungsgemäßen Zubereitungen auch weitere Farbstoffe enthalten. Vorteilhafte Farbpigmente sind beispielsweise Titandioxid, Glimmer, Eisenoxide (z. B. Fe₂O₃, Fe₃O₄, FeO(OH)) und/oder Zinnoxid. Vorteilhafte Farbstoffe sind beispielsweise Carmin, Berliner Blau, Chromoxidgrün, Ultramarinblau und/oder Manganviolett. Es ist insbesondere vorteilhaft, die Farbstoffe und/oder Farbpigmente aus der folgenden Liste zu wählen. Die Colour Index Nummern (CIN) sind dem *Rowe Colour Index, 3. Auflage, Society of Dyers and Colourists, Bradford, England, 1971* entnommen.

| Chemische oder sonstige Bezeichnung | CIN | Farbe |
|---|---|---|
| Pigment Green | 10006 | Grün |
| Acid Green 1 | 10020 | Grün |
| 2,4-Dinitrohydroxynaphthalin-7-sulfosäure | 10316 | Gelb |
| Pigment Yellow 1 | 11680 | Gelb |
| Pigment Yellow 3 | 11710 | Gelb |
| Pigment Orange 1 | 11725 | Orange |
| 2,4-Dihydroxyazobenzol | 11920 | Orange |
| Solvent Red 3 | 12010 | Rot |
| 1-(2'-Chlor-4'-nitro-1'-phenylazo)-2-hydroxynaphthalin | 12085 | Rot |
| Pigment Red 3 | 12120 | Rot |
| Ceresrot; Sudanrot; Fettrot G | 12150 | Rot |
| Pigment Red 112 | 12370 | Rot |
| Pigment Red 7 | 12420 | Rot |
| Pigment Brown 1 | 12480 | Braun |
| 4-(2'-Methoxy-5'-sulfosäurediethylamid-1'-phenylazo)-3-hydroxy-5"-chloro-2",4"-dimethoxy-2-naphthoesäureanilid | 12490 | Rot |
| Disperse Yellow 16 | 12700 | Gelb |
| 1-(4-Sulfo-1-phenylazo)-4-amino-benzol-5-sulfosäure | 13015 | Gelb |
| 2,4-Dihydroxy-azobenzol-4'-sulfosäure | 14270 | Orange |
| 2-(2,4-Dimethylphenylazo-5-sulfosäure )-1-hydroxynaphthalin-4-sulfosäure | 14700 | Rot |
| 2-(4-Sulfo-1-naphthylazo)-1-naphthol-4-sulfosäure | 14720 | Rot |
| 2-(6-Sulfo-2,4-xylylazo)-1-naphthol-5-sulfosäure | 14815 | Rot |
| 1-(4'-Sulfophenylazo)-2-hydrooynaphthalin | 15510 | Orange |
| 1-(2-Sulfosäure-4-chlor-5-carbonsäure-1-phenylazo)-2-hydroxynaphthalin | 15525 | Rot |
| 1-(3-Methyl-phenylazo-4-sulfosäure)-2-hydroxynaphthalin | 15580 | Rot |
| 1-(4',(8')-Sulfosäurenaphthylazo)-2-hydroxynaphthalin | 15620 | Rot |
| 2-Hydrooy-1,2'-azonaphthalin-1'-sulfosäure | 15630 | Rot |
| 3-Hydroxy-4-phenylazo-2-naphthylcarbonsäure | 15800 | Rot |
| 1-(2-Sulfo-4-methyl-1-phenylazo)-2-naphthylcarbonsäure | 15850 | Rot |
| 1-(2-Sulfo-4-methyl-5-chlor-1-phenylazo)-2-hydroxynaphthalin-3-carbonsäure | 15865 | Rot |
| 1-(2-Sulfo-1-naphthylazo)-2-hydroxynaphthalin-3-carbonsäure | 15880 | Rot |
| 1-(3-Sulfo-1-phenylazo)-2-naphthol-6-sulfosäure | 15980 | Orange |
| 1-(4-Sulfo-1-phenylazo)-2-naphthol-6-sulfosäure | 15985 | Gelb |
| Allura Red | 16035 | Rot |
| 1-(4-Sulfo-1-naphthylazo)-2-naphthol-3,6-disulfosäure | 16185 | Rot |
| Acid Orange 10 | 16230 | Orange |
| 1-(4-Sulfo-1-naphthylazo)-2-naphthol-6,8-disulfosäure | 16255 | Rot |
| 1-(4-Sulfo-1-naphthylazo)-2-naphthol-3,6,8-trisulfosäure | 16290 | Rot |
| 8-Amino-2 -phenylazo-1-naphthol-3,6-disulfosäure | 17200 | Rot |
| Acid Red 1 | 18050 | Rot |
| Acid Red 155 | 18130 | Rot |
| Acid Yellow 121 | 18690 | Gelb |
| Acid Red 180 | 18736 | Rot |
| Acid Yellow 11 | 18820 | Gelb |
| Acid Yellow 17 | 18965 | Gelb |
| 4-(4-Sulfo-1-phenylazo)-1-(4-sulfophenyl)-5-hydroxypyrazolon-3-carbonsäure | 19140 | Gelb |
| Pigment Yellow 16 | 20040 | Gelb |
| 2,6-(4'-Sulfo-2", 4"-dimethyl)-bis-phenylazo)1,3-dihydroxybenzol | 20170 | Orange |
| Acid Black 1 | 20470 | Schwarz |
| Pigment Yellow 13 | 21100 | Gelb |
| Pigment Yellow 83 | 21108 | Gelb |
| Solvent Yellow | 21230 | Gelb |
| Acid Red 163 | 24790 | Rot |
| Acid Red 73 | 27290 | Rot |
| 2-[4'-(4"-Sulfo-1"-phenylazo)-7'-sulfo-1'-naphthylazo]-1-hydroxy-7-aminonaphthalin-3,6-disulfosäure | 27755 | Schwarz |
| 4'-[(4"-Sulfo-1"-phenylazo)-7'-sulfo-1'-naphthylazo]-1-hydroxy-8-acetyl-aminonaphthalin-3,5-disulfosäure | 28440 | Schwarz |
| Direct Orange 34, 39, 44, 46, 60 | 40215 | Orange |
| Food Yellow | 40800 | Orange |
| trans-ß-Apo-8'-Carotinaldehyd (C₃₀) | 40820 | Orange |
| trans-Apo-8'-Carotinsäure (C₃₀)-ethylester | 40825 | Orange |
| Canthaxanthin | 40850 | Orange |
| Acid Blue 1 | 42045 | Blau |
| 2,4-Disulfo-5-hydroxy-4'-4"-bis-(diethylamino)triphenyl-carbinol | 42051 | Blau |
| 4-[(-4-N-Ethyl-p-sulfobenzylamino)-phenyl-(4-hydroxy-2-sulfophenyl)-(methylen)-1-(N-ethyIN-p-sulfobenzyl)-2,5-cyclohexadienimin] | 42053 | Grün |
| Acid Blue 7 | 42080 | Blau |
| (N-Ethyl-p-sulfobenzyl-amino)-phenyl-(2-sulfophenyl)-methylen-(N-ethyl-N-p-sulfo-benzyl)Δ^{2,5}-cyclohexadienimin | 42090 | Blau |
| Acid Green 9 | 42100 | Grün |
| Diethyl-di-sulfobenzyl-di-4-amino-2-chlor-di-2-methyl-fuchsonimmonium | 42170 | Grün |
| Basic Violet 14 | 42510 | Violett |
| Basic Violet 2 | 42520 | Violett |
| 2'-Methyl-4'-(N-ethyl-N-m-sulfobenzyl)-amino-4"-(N-diethyl)- | 42735 | Blau |
| amino-2-methyl-N-ethylN-m-sulfobenzyl-fuchsonimmonium | | |
| 4'-(N-Dimethyl)-amino-4"-(N-phenyl)-aminonaphtho-N-dimethyl-fuchsonimmonium | 44045 | Blau |
| 2-Hydroxy-3,6-disulfo-4,4'-bis-dimethylaminonaphthofuchsonimmonium | 44090 | Grün |
| Acid Red 52 | 45100 | Rot |
| 3-(2'-Methylphenylamino)-6-(2'-methyl-4'-sulfophenylamino)-9-(2"-carboxyphenyl)-xantheniumsalz | 45190 | Violett |
| Acid Red 50 | 45220 | Rot |
| Phenyl-2-oxyfluoron-2-carbonsäure | 45350 | Gelb |
| 4,5-Dibromfluorescein | 45370 | Orange |
| 2,4,5,7-Tetrabromfluorescein | 45380 | Rot |
| Solvent Dye | 45396 | Orange |
| Acid Red 98 | 45405 | Rot |
| 3',4',5',6'-Tetrachlor-2,4,5,7-tetrabromfluorescein | 45410 | Rot |
| 4,5-Diiodfluorescein | 45425 | Rot |
| 2,4,5,7-Tetraiodfluorescein | 45430 | Rot |
| Chinophthalon | 47000 | Gelb |
| Chinophthalon-disulfosäure | 47005 | Gelb |

Es kann ferner günstig sein, als Farbstoff eine oder mehrer Substanzen aus der folgenden Gruppe zu wählen: 2,4-Dihydroxyazobenzol, 1-(2'-Chlor-4'-nitro-1'-phenylazo)-2-hydroxynaphthalin, Ceresrot, 2-(4-Sulfo-1-naphthylazo)-1-naphthol-4-sulfosäure, Calciumsalz der 2-Hydroxy-1,2'-azonaphthalin-1'-sulfosäure, Calcium- und Bariumsalze der 1-(2-Sulfo-4-methyl-1-phenylazo)-2-naphthylcarbonsäure, Calciumsalz der 1-(2-Sulfo-1-naphthylazo)-2-hydroxynaphthalin-3-carbonsäure, Aluminiumsalz der 1-(4-Sulfo-1-phenylazo)-2-naphthol-6-sulfosäure, Aluminiumsalz der 1-(4-Sulfo-1-naphthylazo)-2-naphthol-3,6-disulfosäure, 1-(4-Sulfo-1-naphthylazo)-2-naphthol-6,8-disulfosäure, Aluminiumsalz der 8-Amino-2 - phenylazo- 1-naphthol-3,6-disulfosäure , Aluminiumsalz der 4-(4-Sulfo-1-phenylazo)-1-(4-sulfophenyl)-5-hydroxy-pyrazolon-3-carbonsäure, 4'-[(4"-Sulfo-1 "-phenylazo)-7'-sulfo-1'-naphthylazo]-1-hydroxy-8-acetyl-aminonaphthalin-3,5-disulfosäure, Aluminium- und Zirkoniumsalze von 4,5-Dibromfluorescein, Aluminium- und Zirkoniumsalze von 2,4,5,7-Tetrabromfluorescein, 3',4',5',6'-Tetrachlor-2,4,5,7-tetrabromfluorescein und sein Aluminiumsalz, Aluminiumsalz von 2,4,5,7-Tetraiodfluorescein, Aluminiumsalz der Chinophthalon-disulfosäure, Aluminiumsalz der Indigo-disulfosäure, 4,4'-Dimethyl-6,6'-dichlorthioindigo, Komplexsalz (Na, Al, Ca) der Karminsäure, rotes und schwarzes Eisenoxid (CIN: 77 491 (rot) und 77 499 (schwarz)), Eisenoxidhydrat (CIN: 77 492), Manganammoniumdiphosphat (CIN 77745), Ultramarin (CIN 77007) und Titandioxid.

Erfindungsgemäße Titandioxide, die sowohl in der Kristallmodifikation Rutil als auch Anatas vorliegen können, sind im Sinne der vorliegenden Erfindung vorteilhaft oberflächlich behandelt ("gecoatet"), wobei beispielsweise ein hydrophiler, amphiphiler oder hydrophober Charakter gebildet werden bzw. erhalten bleiben soll. Diese Oberflächenbehandlung kann darin bestehen, dass die Pigmente nach an sich bekannten Verfahren mit einer dünnen hydrophilen und/oder hydrophoben anorganischen und/oder organischen Schicht versehen werden. Die verschiedenen Oberflächenbeschichtungen können im Sinne der vorliegenden Erfindung auch Wasser enthalten.

Anorganische Oberflächenbeschichtungen im Sinne der vorliegenden Erfindung können bestehen aus Aluminiumoxid (Al₂O₃), Aluminiumhydroxid Al(OH)₃, bzw. Aluminiumoxidhydrat (auch: Alumina, CAS-Nr.: 1333-84-2), Natriumhexametaphosphat (NaPO₃)₆, Natriummetaphosphat (NaPO₃)ₙ, Siliciumdioxid (SiO₂) (auch: Silica, CAS-Nr.: 7631-86-9), Zirkoniumoxid (ZrO₂) oder Eisenoxid (Fe₂O₃). Diese anorganischen Oberflächenbeschichtungen können allein, in Kombination und/oder in Kombination mit organischen Beschichtungsmaterialien vorkommen.
Hierzu werden Oxide, Oxidhydrate oder Phosphate beispielsweise der Elemente Al, Si, Zr in dichten Schichten auf die Pigmentoberfläche aufgefällt.
Die anorganische Nachbehandlung geschieht im allgemeinen in einer wässrigen Suspension des Pigmentes durch Zugabe löslicher Nachbehandlungschemikalien, wie z.B. Aluminiumsulfat, und anschließende Ausfällung des im neutralen Bereich schwerlöslichen Hydroxides durch gezielte Einstellung des pH-Wertes mit Natronlauge.
Nach der anorganischen Nachbehandlung werden die gecoateten Pigmente durch Filtration aus der Suspension abgetrennt und sorgfältig gewaschen, um die gelösten Salze zu entfernen, anschließend werden die isolierten Pigmente getrocknet.

Besonders bevorzugt im Sinne dieser Erfindung sind Titandioxide, auf die Aluminiumhydroxid auf die Oberfläche aufgebracht worden ist, wie z.B. die von Sun Chemical erhältlichen Titandioxid Typen C47-051 und C47- 5175. Weiterhin bevorzugte Pigmente sind Titandioxide, die mit Aluminium- und / oder Siliziumoxiden gecoated sind, wie z.B. von der Firma Krosnos Titan: Kronos 1071 und 1075 oder von der Firma Kingfisher: A310.03 Tudor Aspen.

Organische Oberflächenbeschichtungen im Sinne der vorliegenden Erfindung können bestehen aus pflanzlichem oder tierischem Aluminiumstearat, pflanzlicher oder tierischer Stearinsäure, Laurinsäure, Dimethylpolysiloxan (auch: Dimethicone), Methylpolysiloxan (Methicone), Simethicone (einem Gemisch aus Dimethylpolysiloxan mit einer durchschnittlichen Kettenlänge von 200 bis 350 Dimethylsiloxan-Einheiten und Silicagel) oder Alginsäure. Diese organischen Oberflächenbeschichtungen können allein, in Kombination und/oder in Kombination mit anorganischen Beschichtungsmaterialien vorkommen.

Ferner kann es erfindungsgemäß vorteilhaft sein Perlglanzpigmente einzusetzen.
Dazu zählen natürliche Perlglanzpigmente, wie z. B.
■ "Fischsilber" (Guanin/Hypoxanthin-Mischkristalle aus Fischschuppen) und
■ "Perlmutt" (vermahlene Muschelschalen),
monokristalline Perlglanzpigmente wie z. B. Bismuthoxychlorid (BiOCI), Schicht-Substrat Pigmente: z. B. Glimmer / Metalloxid

Basis für Perlglanzpigmente sind beispielsweise pulverförmige Pigmente oder Ricinusöldispersionen von Bismutoxychlorid und/oder Titandioxid sowie Bismutoxychlorid und/oder Titandioxid auf Glimmer. Insbesondere vorteilhaft ist z. B. das unter der CIN 77163 aufgelistete Glanzpigment.

Vorteilhaft sind ferner beispielsweise die folgenden Perlglanzpigmentarten auf Basis von Glimmer/Metalloxid:

| ***Gruppe*** | Belegung / Schichtdicke | Farbe |
|---|---|---|
| **Silberweiße Perlglanzpigmente** | TiO₂: 40 - 60 nm | silber |
| **Interferenzpigmente** | TiO₂: 60 - 80 nm | gelb |
| | TiO₂: 80 -100 nm | rot |
| | TiO₂: 100 - 140 nm | blau |
| | TiO₂: 120 - 160 nm | grün |
| **Farbglanzpigmente** | Fe₂O₃ | bronze |
| | Fe₂O₃ | kupfer |
| | Fe₂O₃ | rot |
| | Fe₂O₃ | rotviolett |
| | Fe₂O₃ | rotgrün |
| | Fe₂O₃ | schwarz |
| **Kombinationspigmente** | TiO₂ / Fe₂O₃ | Goldtöne |
| | TiO₂ / Cr₂O₃ | grün |
| | TiO₂ / Berliner Blau | tiefblau |
| | TiO₂ / Carmin | rot |

Besonders bevorzugt sind die von der Firma Merck unter den Handelsnamen Timiron, Colorona oder Dichrona erhältlichen Perlglanzpigmente.

Die Liste der genannten Perlglanzpigmente soll selbstverständlich nicht limitierend sein. Im Sinne der vorliegenden Erfindung vorteilhafte Perlglanzpigmente sind auf zahlreichen, an sich bekannten Wegen erhältlich. Beispielsweise lassen sich auch andere Substrate außer Glimmer mit weiteren Metalloxiden beschichten, wie z. B. Silica und dergleichen mehr. Vorteilhaft sind z. B. mit TiO₂ und Fe₂O₃ beschichtete SiO₂-Partikel ("Ronaspheren"), die von der Firma Merck vertrieben werden.

Es kann darüber hinaus von Vorteil sein, gänzlich auf ein Substrat wie Glimmer zu verzichten. Besonders bevorzugt sind Perlglanzpigmente, welche unter der Verwendung von SiO₂ hergestellt werden. Solche Pigmente, die auch zusätzlich gonichromatische Effekte haben können, sind z. B. unter dem Handelsnamen Sicopearl Fantastico bei der Firma BASF erhältlich.

Weiterhin vorteilhaft können Pigmente der Firma Engelhard / Mearl auf Basis von Calcium Natrium Borosilikat, die mit Titandioxid beschichtet sind, eingesetzt werden. Diese sind unter dem Namen Reflecks erhältlich. Sie weisen durch ihrer Partikelgröße von 40 - 180 µm zusätzlich zu der Farbe einen Glitzereffekt auf.

Die Farbstoffe und Pigmente können sowohl einzeln als auch im Gemisch vorliegen sowie gegenseitig miteinander beschichtet sein, wobei durch unterschiedliche Beschichtungsdicken im allgemeinen verschiedene Farbeffekte hervorgerufen werden.

Die erfindungsgemäß besonders bevorzugten pigmentären Farbstoffe sind die aufgeführten blauen Pigmente wie z.B. INCI: CI 77007, Outremer Supercosmetique W 6803 von Les Colorants Wackherr, INCI: CI 77891 + Mica + Silica, Timiron Splendid Blue von Merck.

Erfindungsgemäß bevorzugt sind ferner mit Titandioxid und Siliciumdioxid beschichteter Glimmer, z.B. INCI: Mica + CI 77891 + Silica, Timiron Arctic Silver von Merck, INCI: Mica + CI 77891, Timiron Gleamer Flake MP-45 von Merck; mit Titandioxid und Zinnoxid beschichtetes Aluminiumoxid oder Siliciumdioxid, z.B.INCI: Silica + CI 77891 + Tin Oxide, Xirona Magic Mauve von Merck; z.B.INCI: Alumina + CI 77891 + Tin Oxide, Xirona Silver, mit Titandioxid und Berliner Blau bschichteter Glimmer, z.B. INCI: Mica + CI 77891 + CI 77510, Colorona Light Blue und Colorona Dark Blue von Merck.

Auch Glitter- und Glitzerpartikel können erfindungsgemäß vorteilhaft in mindestens einer der Zubereitungen enthalten sein.
Erfindungsgemäß vorteilhaft können auch anionische, kationische, nichtionische und zwitterionische Tenside in den Zubereitungen enthalten sein.

### Komplexbildner

Es ist auch von Vorteil, den erfindungsgemäßen Zubereitungen Komplexbildner zuzusetzen. Vorteilhaft werden die Komplexbildner gewählt aus der Gruppe bestehend aus Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen, Tetrasodium Iminodisuccinate, Trisodium Etylenediamine Disuccinate.

### Vergleichsversuch

Die erfindungsgemäß vorteilhaften Eigenschaften, die bei der erfindungsgemäßen Verwendung von hydrierten Sojaglyceriden zu beobachten sind. Ließen sich beispielsweise mit dem folgenden Vergleichsversuch belegen:
Die Versuche wurden von 10 Versuchspersonen durchgeführt, die sich jeweils eine definierte Menge (0,1 ml) an Tagescreme der Rezepturen I und II in jeweils der gleichen Weise auf jeweils einen Unterarm auftrugen, die Creme verteilten und nach dem Eincremen den sensorischen Eindruck beurteilten.

| **Formelgrundlagen** | **Rezeptur I** | **Rezeptur II** |
|---|---|---|
| | | mit 2% |
| | | hydriertem |
| | mit 2% Shea | Sojaglyceriden |
| | Butter (INCI: | (INCI: |
| Nivea Visage Tagescreme | Butyrospermum | Hydrogenated |
| | Parkii) | Vegetable Oil) |

### Ergebnisse:

1. Sensorische Beurteilung von 10 Personen (Halbseitenvergleich am Unterarm)

| **Sensorikkriterien** | **Rezeptur I** | **Rezeptur II** |
|---|---|---|
| | | Sehr gut |
| Verteilbarkeit | verteilbar | verteilbar |
| | stumpf, | Weich, |
| Rückstand | wachsartig | geschmeidig |

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

### Beispiele

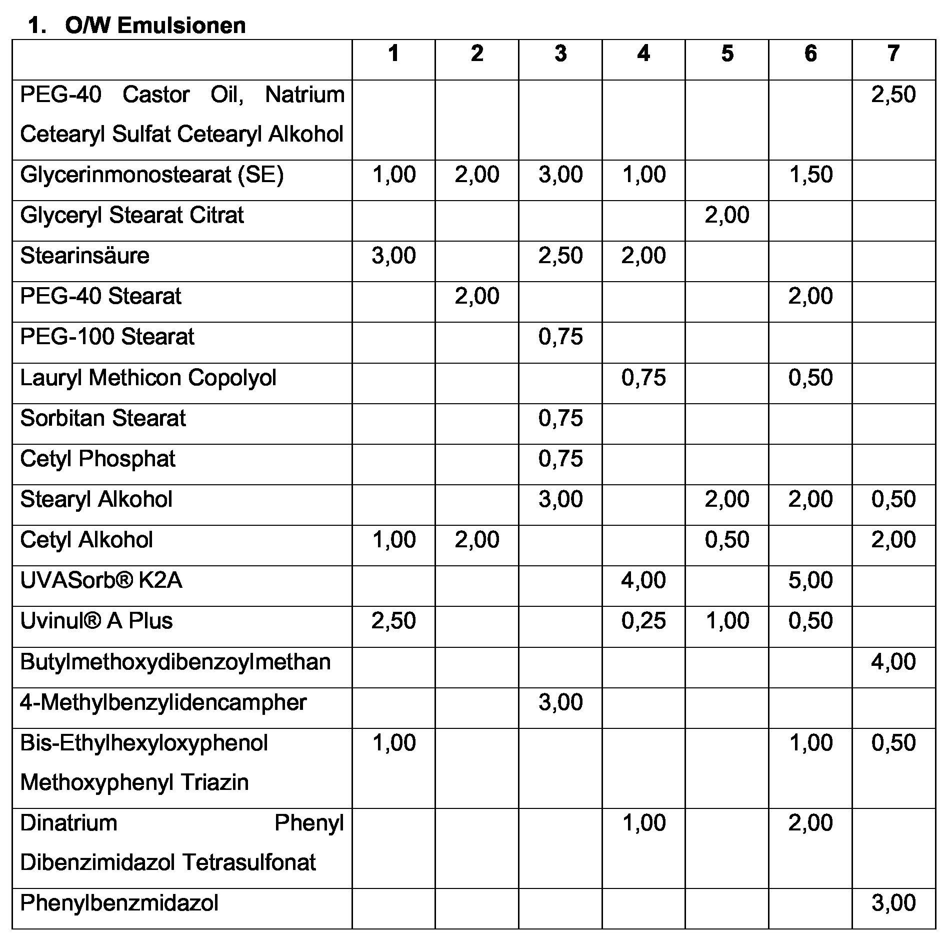

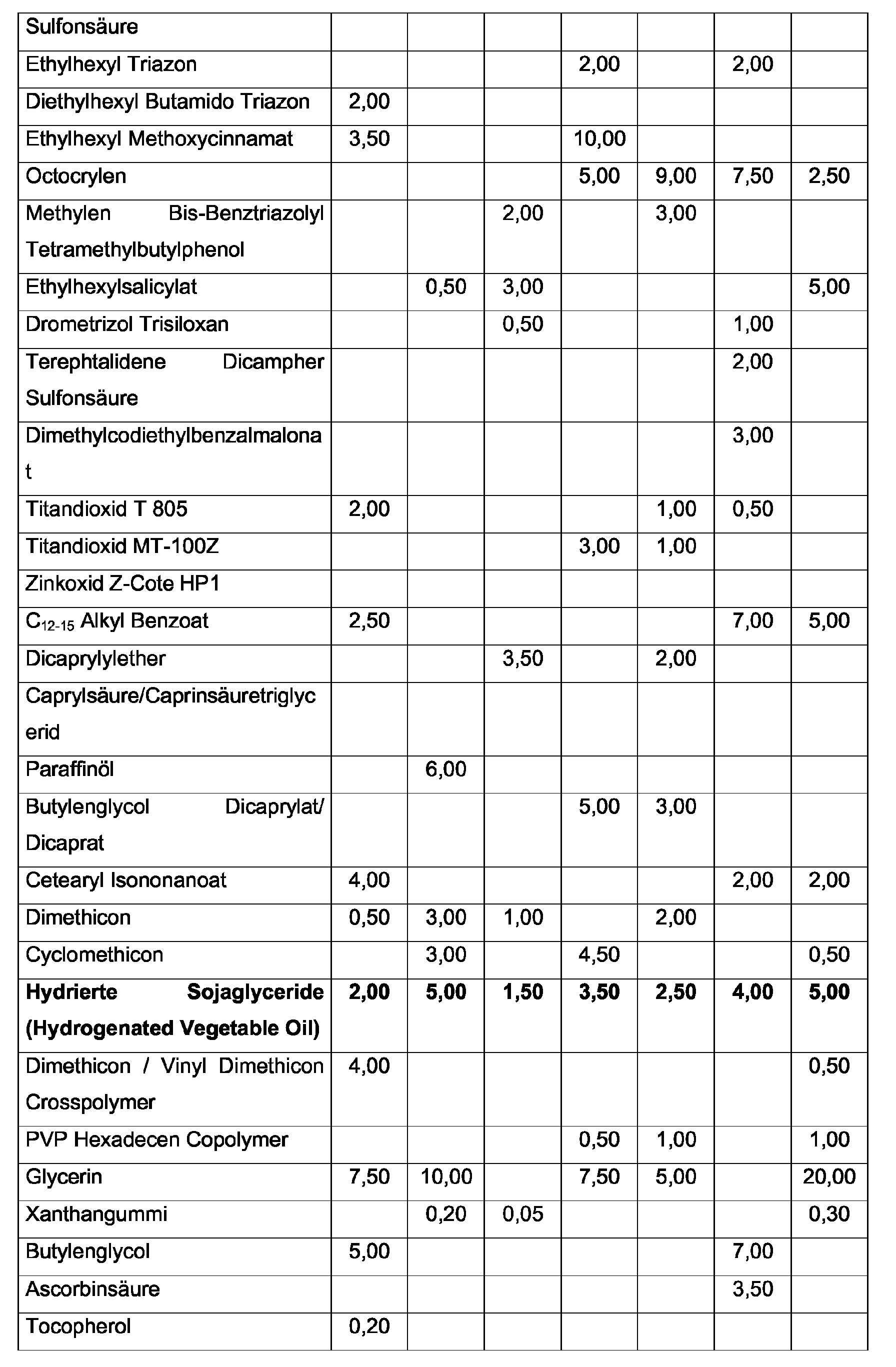

| | | | | | | | |
|---|---|---|---|---|---|---|---|
| Taurin | 1,00 | | | | | | |
| Vitamin E Acetat | | 0,40 | 0,25 | 0,50 | 0,75 | | 1,00 |
| Retinol | | | | | 0,05 | | |
| Dioic Acid (Octadecendicarbonsäure) | | | | 0,20 | | 0,25 | |
| Pyridoxin | | 0,5 | | | | | |
| Fucogel® 1000 | | | 1,50 | | | 5,00 | |
| Dihydroxyaceton | | 5,00 | | | | | |
| DMDM Hydantoin | 0,60 | | 0,40 | 0,20 | | | |
| Methylparaben | | 0,10 | 0,25 | | 0,50 | | |
| Phenoxyethanol | 0,40 | 0,50 | | 0,40 | 0,50 | | 0,60 |
| EDTA | 0,20 | | 0,35 | 0,50 | 0,02 | | 0,03 |
| Ethanol | 2,00 | | 1,50 | | 3,00 | 5,00 | 1,00 |
| Insekt Repellent 3535 | | | 5,00 | | | | |
| Parfüm | 0,20 | 0,20 | | | | 0,30 | 0,40 |
| Wasser | ad 100 | ad 100 | ad 100 | ad 100 | ad 100 | ad 100 | ad 100 |
| Neutralisationsmittel (Natriumhydroxid, Kaliumhydroxid) | qs | qs | qs | qs | qs | qs | qs |
| pH-Wert | 6,0-7,5 | 4,5-7,0 | 6,5-8,5 | 5,0-7,0 | 6,0-8,0 | 4,0-6,0 | 5,0-7,5 |

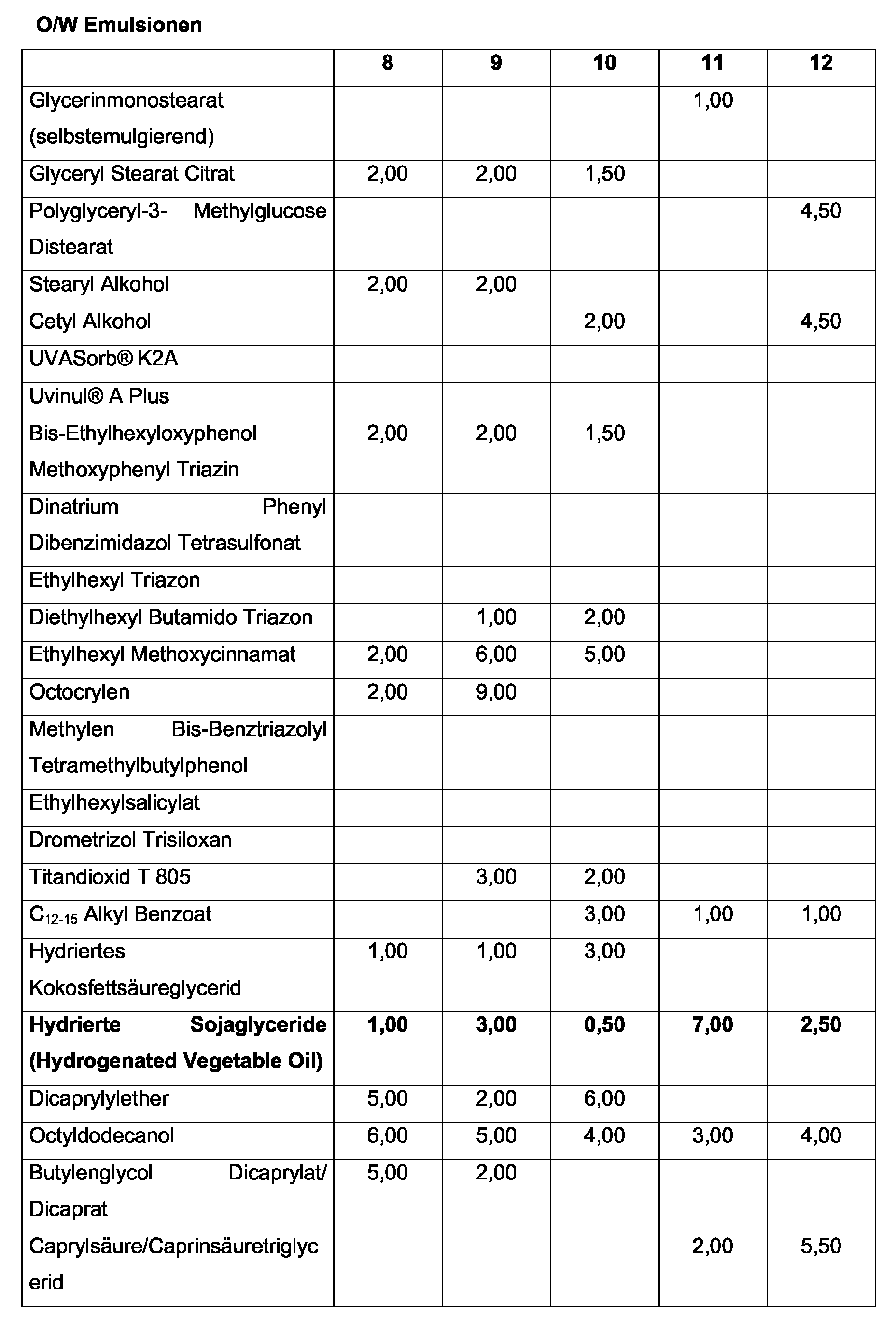

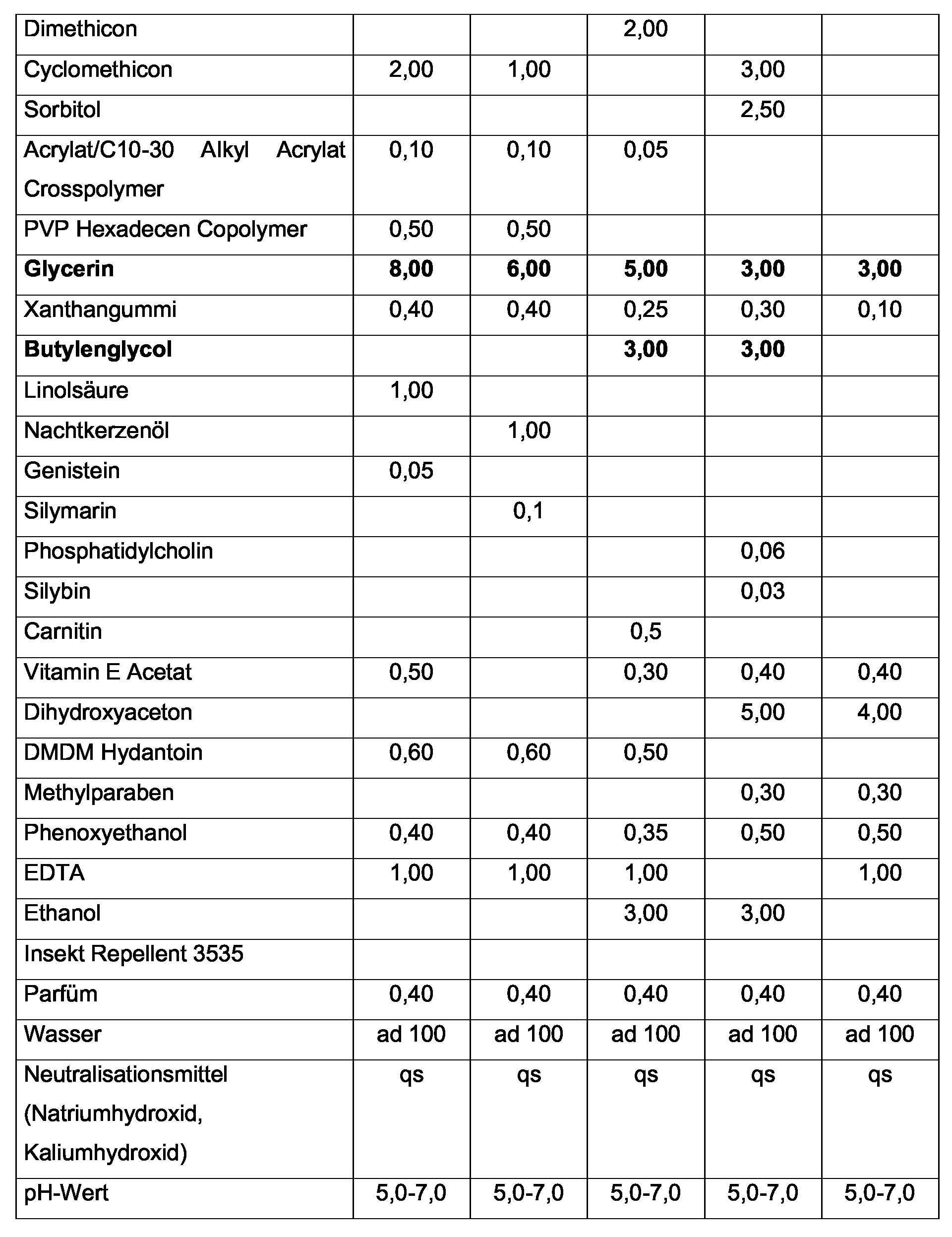

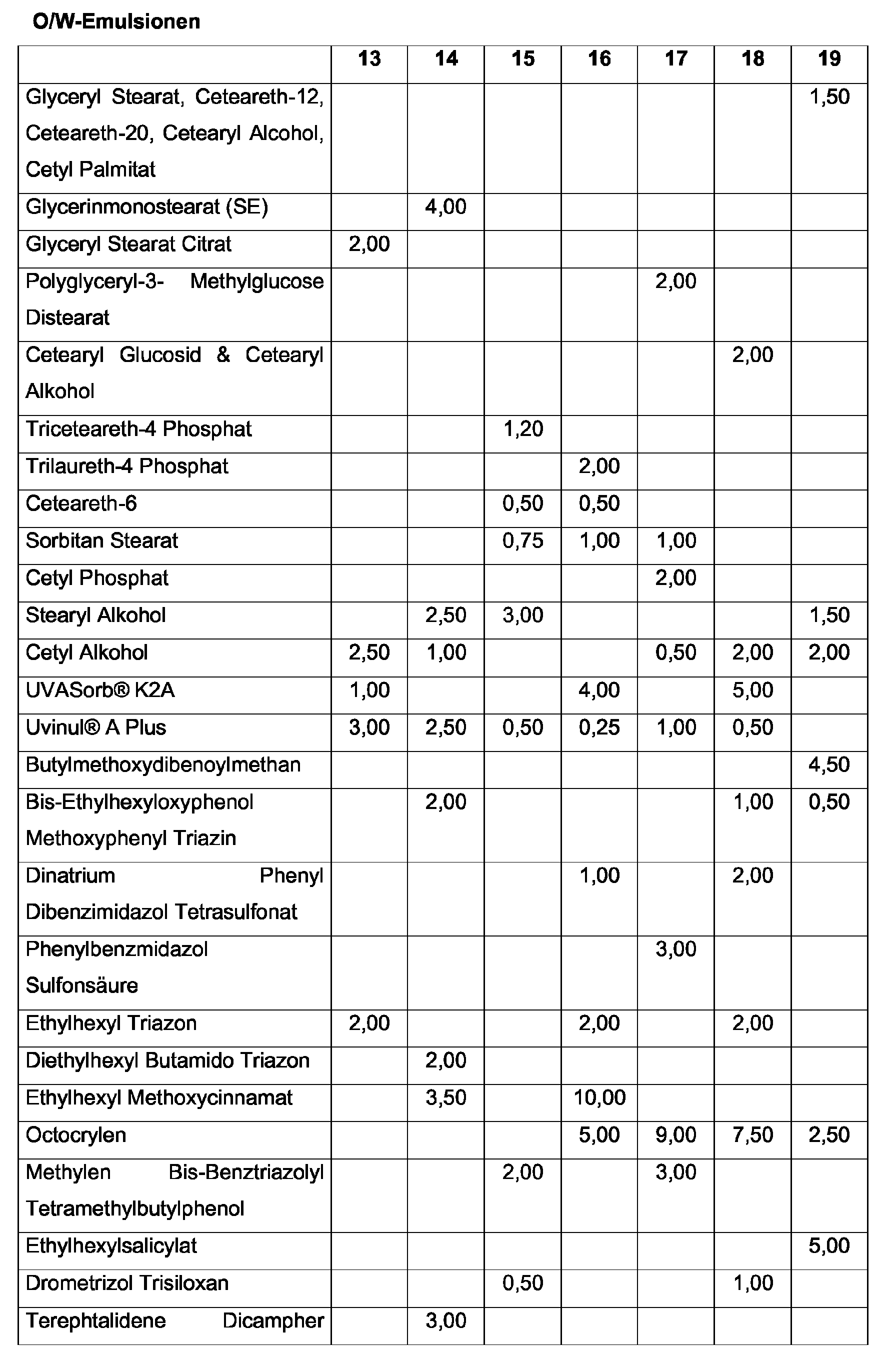

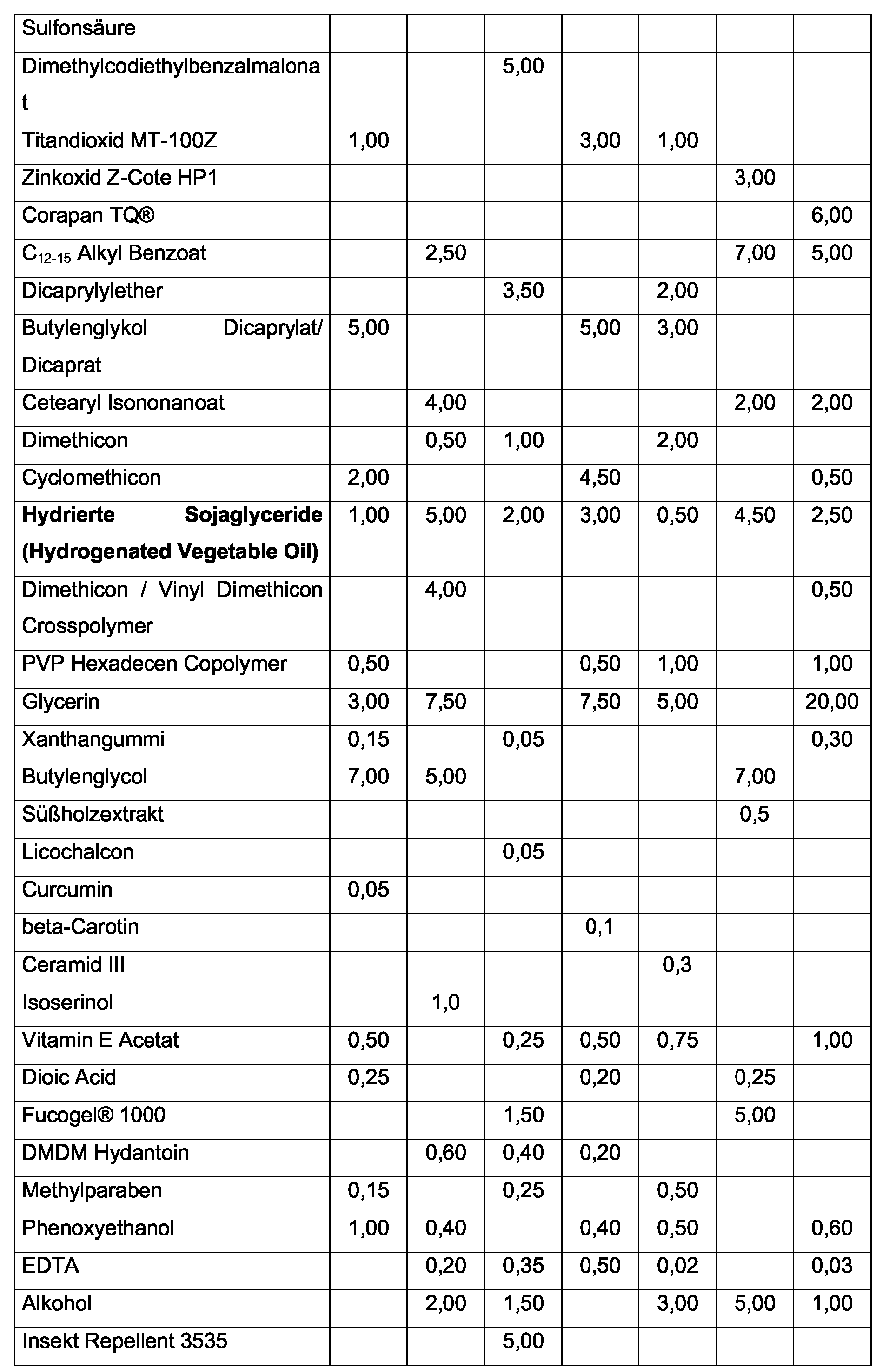

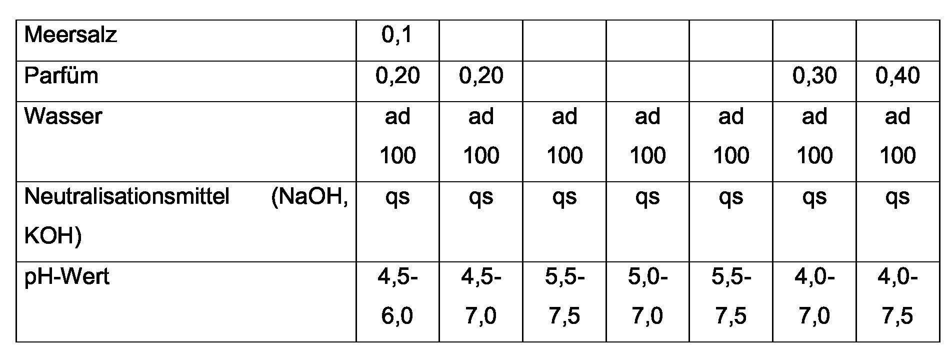

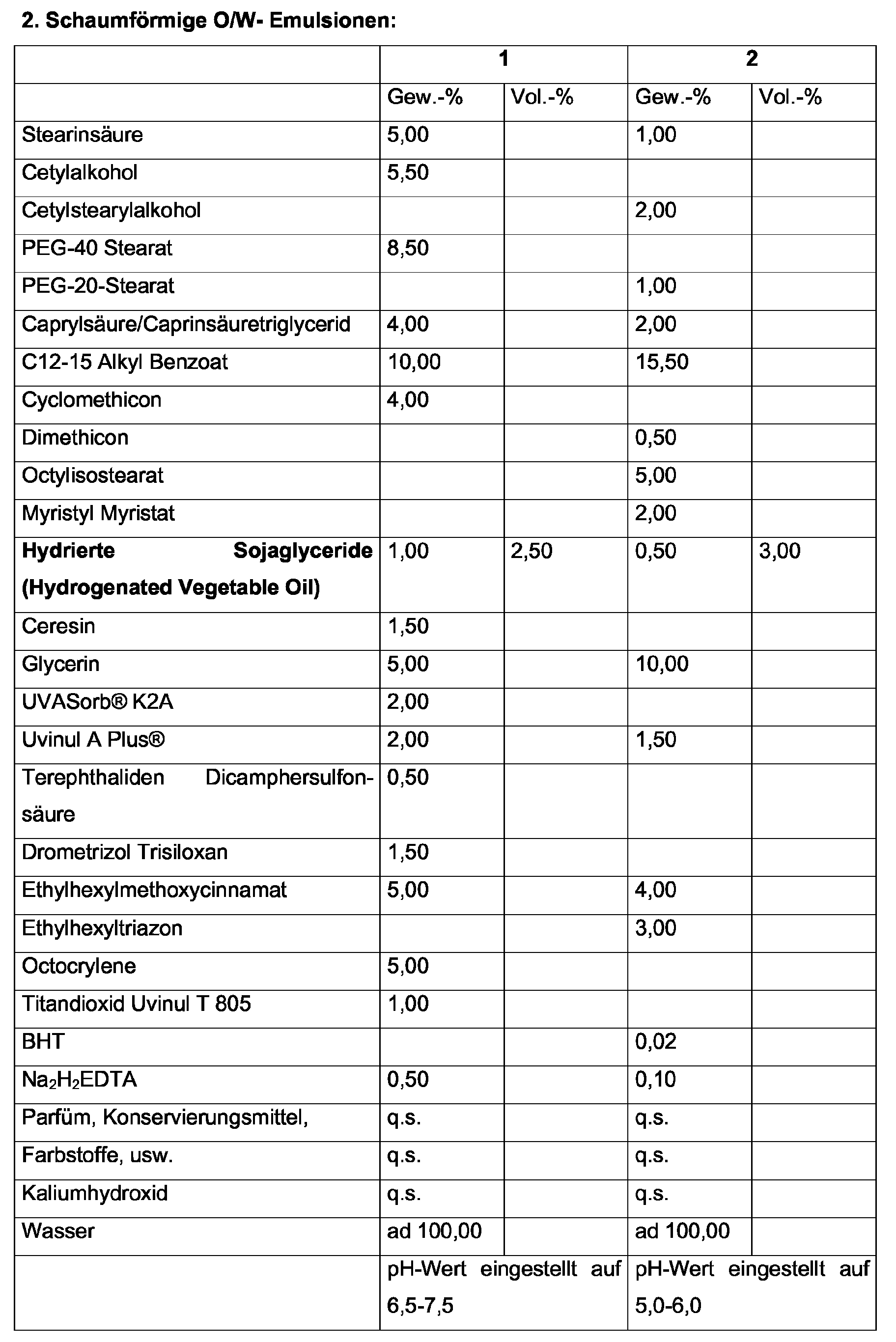

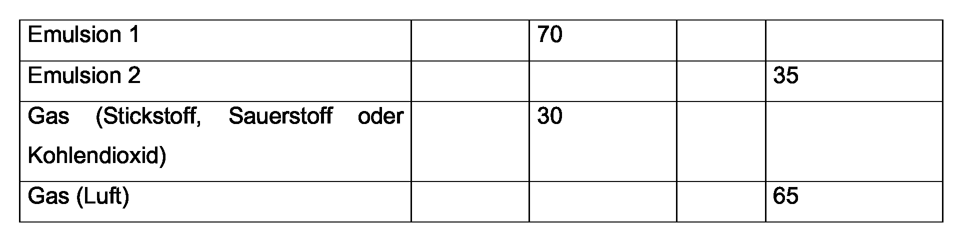

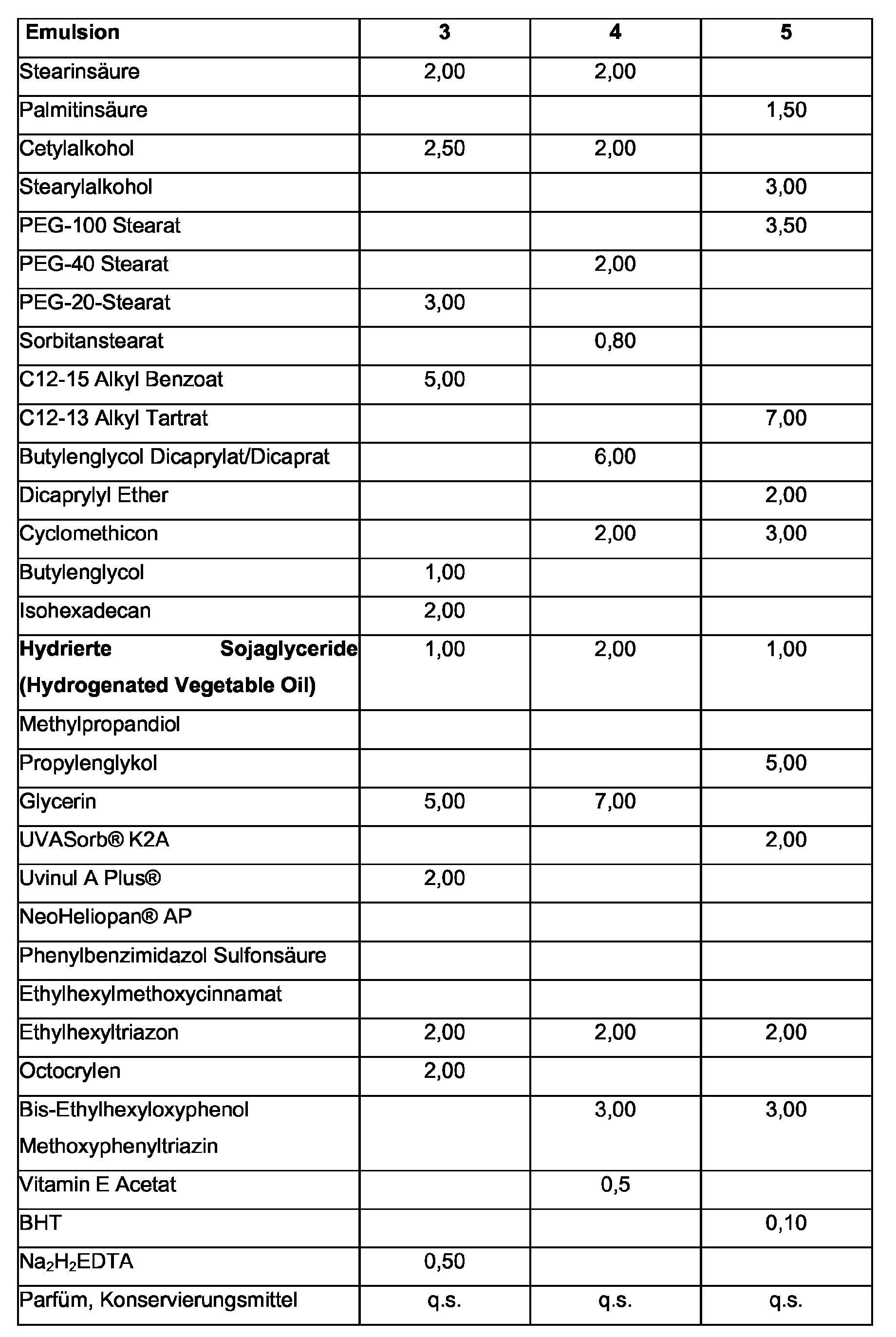

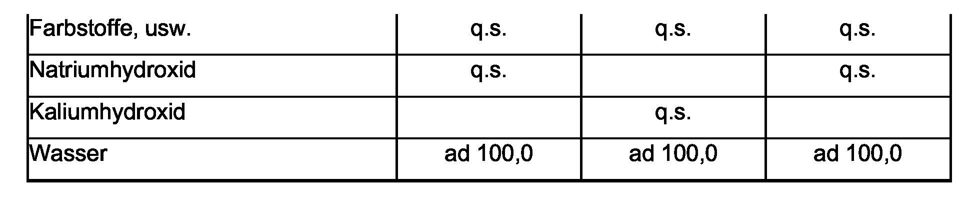

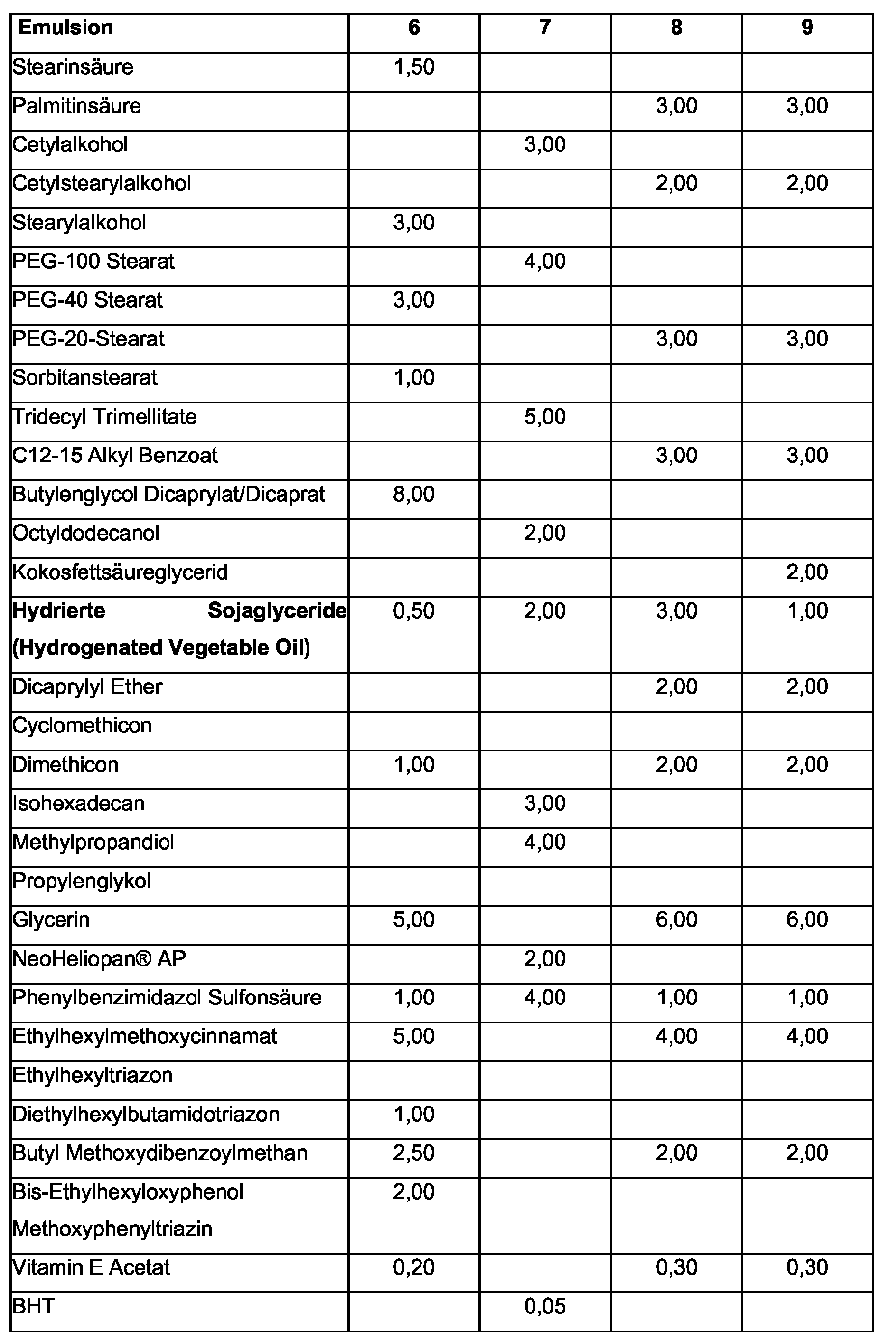

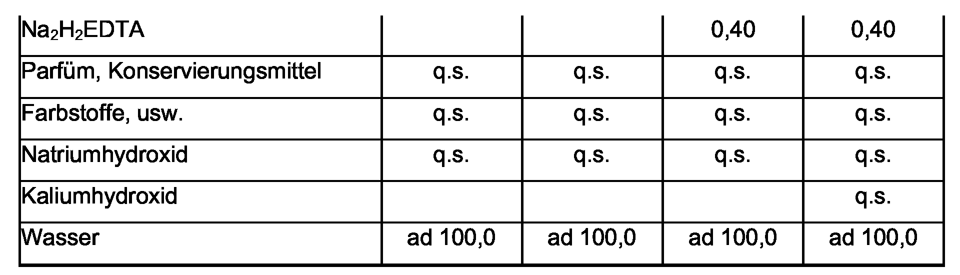

Zur Herstellung des Schaums werden 80-97 Vol.-% der Emulsion I mit 3-20 Vol.-% eines geeigneten Gases (z.B. Propan/ Butan, Druckluft, Stickstoff) aufgeschäumt.

| **3. W/O-Emulsionen** | | | | | |
|---|---|---|---|---|---|
| | **1** | **2** | **3** | **4** | **5** |
| Cetyl Dimethicon Copolyol | 4,00 | | | 2,50 | 3,00 |
| Polyglyceryl-2 Dipolyhydroxystearat | | | 3,00 | | 1,00 |
| Isostearyl Diglyceryl Succinat | | | 0,75 | | 0,30 |
| Lauryl Methicon Copolyol | | | | 2,00 | |
| Polysorbat-65 | | | 2,00 | | 1,50 |
| PEG-100 Stearat | | | | 1,20 | 0,70 |
| Cetearyl Sulfat | | | 0,25 | | 1,00 |
| Dimethicon | | 4,00 | | | 2,00 |
| Cyclomethicon | 12,00 | 20,00 | | 30,00 | 15,00 |
| UVASorb® K2A | | | | 0,50 | |
| Uvinul® A Plus | | 2,00 | 0,50 | 4,00 | 0,25 |
| Bis-Ethylhexyloxyphenol Methoxyphenyl Triazin | 1,00 | | | 0,50 | |
| Dinatrium Phenyl Dibenzimidazol Tetrasulfonat | | 1,50 | | | 2,00 |
| Drometrizol Trisiloxan | | | | 1,00 | |
| 4-Methylbenzylidencampher | 4,00 | | | | |
| Methylen Bis-Benztriazolyl Tetramethylbutylphenol | | | | | 3,00 |
| Ethylhexyl Methoxycinnamat | 3,00 | 4,00 | | | 10,00 |
| Ethylheyl Salicylat | | | 5,00 | | 3,50 |
| Octocrylen | | 5,00 | | 4,00 | |
| Diethylhexyl Butamido Triazon | | 1,00 | | | 6,50 |
| Ethylhexyl Triazon | 3,00 | | | | 4,00 |
| Titandioxid MT-100 Z | | 0,50 | 1,00 | 1,50 | 0,50 |
| Zinkoxid Z-Cote | 2,00 | | | | 4,00 |
| Dicaprylyl Carbonat | 5,00 | | 15,00 | | 4,00 |
| Dihexyl Carbonat | | 10,00 | | | |
| C12-15 Alkyl Benzoat | 7,00 | | 10,00 | | |
| Mineral Öl | 10,00 | | | | 6,00 |
| Kokosfettsäureglycerid | | 2,00 | | 5,00 | |
| **Hydrierte Sojaglyceride (Hydrogenated Vegetable Oil)** | 2,00 | 1,00 | 5,00 | 0,50 | 7,00 |
| PVP Hexadecen Copolymer | | 0,75 | | | 0,40 |
| Glycerin | 5,00 | 12,50 | | 5,00 | 15,50 |
| Sorbitol | 5,00 | | 10,00 | | |
| α-Glucosylrutin | | | | | 0,15 |
| EDTA | | 0,15 | 0,03 | | 0,15 |
| Glycin Soja | 0,75 | | | 1,50 | |
| Magnesiumsulfat | 0,75 | 1,00 | | 0,45 | 1,00 |
| DMDM Hydantoin | | 0,05 | | | 0,10 |
| Phenoxyethanol | 1,00 | 0,75 | 0,50 | | 1,00 |
| Alkohol | 2,00 | | | 5,00 | 1,00 |
| Farbstoff, öllöslich | 0,02 | | | | |
| Parfüm | 0,30 | 0,45 | 0,35 | | 0,15 |
| Wassser | ad. 100 | ad. 100 | ad. 100 | ad. 100 | ad. 100 |

| **5.W/O Emulsionen (Cremes & Lotions)** | | | | | | |
|---|---|---|---|---|---|---|
| | **1** | **2** | **3** | **4** | **5** | **6** |
| Cetyldimethicon Copolyol | | | | | 4,00 | |
| Polyglyceryl-2-dipolyhydroxystearat | 5,00 | 4,50 | | | | 4,50 |
| Polyglyceryl-3-Diisostearat | 1,75 | 1,50 | | | | |
| PEG-30-dipolyhydroxystearat | | | 3,00 | 5,00 | | |
| Bis-Ethylhexyloxyphenol Methoxyphenyl Triazin | | | 3,00 | 1,50 | | |
| Butyl Methoxydibenzoylmethan | | | 4,00 | | | |
| UVASorb® K2A | | | | 2,00 | 2,50 | |
| Uvinul ® A Plus | 3,00 | 1,00 | | 3,00 | 0,25 | 2,50 |
| Phenylbenzmidazol Sulfonsäure | | 4,00 | | | 2,00 | 0,50 |
| Ethylhexyl Methoxycinnamat | | 8,00 | | | 5,00 | 4,00 |
| Diethylhexyl Butamido Triazon | 3,00 | 1,00 | | 1,00 | | 3,00 |
| Ethylhexyl Triazon | | | 4,00 | 2,00 | 4,00 | |
| Octocrylen | 7,00 | | | | | 2,50 |
| Drometrizol Trisiloxan | | | | | | |
| Titandioxid Uvinul® T 805 | 2,00 | 1,00 | 5,00 | | | |
| Titandioxid MT-100 TV | | | | | | 2,00 |
| Zinkoxid Z-Cote® HP1 | 2,50 | | | 3,00 | | |
| Corapan TQ® | | | 6,00 | | | |
| Mineralöl | | | 6,00 | 5,00 | | 8,00 |
| Kokosfettsäureglycerid | 4,00 | 6,50 | | 3,00 | | |
| **Hydrierte Sojaglyceride (Hydrogenated Vegetable Oil)** | 1,00 | 0,50 | 7,00 | 1,00 | 2,50 | 3,00 |
| C12-15 Alkyl Benzoat | | | 9,00 | 8,00 | 9,00 | |
| Dicaprylylether | 10,00 | | | | | 7,00 |
| Butylenglycol Dicaprylat/Dicaprat | | | 9,00 | 7,00 | 8,00 | 4,00 |
| Cyclomethicon | 2,00 | | | | | 2,00 |
| PVP Eicosene Copolymer | 0,50 | | | | 1,50 | 1,00 |
| Trinatrium EDTA | 1,00 | | 1,00 | 0,50 | 0,35 | |
| Ethylhexyloxyglycerin | | 0,30 | | | | 0,50 |
| Methylpropandiol | | | | | | 7,50 |
| Glycerin | 5,00 | 7,50 | 6,00 | 8,00 | 7,50 | 2,50 |
| Butylenglykol | | 2,50 | | | | |
| Glycin Soja | | 1,00 | | | | |
| MgSO₄ | 1,00 | 0,50 | 0,30 | 0,30 | 0,50 | |
| Milchsäure & Natriumsalz der Milchsäure | 1,00 | 0,50 | | | | 0,85 |
| Vitamin E | 0,50 | | 0,50 | 1,00 | | 1,00 |
| DMDM Hydantoin | | 0,60 | | | 0,20 | |
| Methylparaben | 0,50 | | | | 0,15 | |
| Phenoxyethanol | 0,50 | 0,40 | 0,50 | 0,60 | 1,00 | 0,60 |
| Dihydroxyaceton | | | | | 5,50 | |
| Alcohol | 3,00 | | 2,00 | 3,00 | | 1,00 |
| Parfüm | 0,20 | | 0,20 | 0,20 | | 0,20 |
| Wasser | ad 100 | ad 100 | ad 100 | ad 100 | ad 100 | ad 100 |

| **6. Hydrodispersionen** | | | | | | |
|---|---|---|---|---|---|---|
| | **1** | **2** | **3** | **4** | **5** | **6** |
| | | | | | | |
| Glyceryl Stearat Citrat | | 0,40 | | | | |
| Cetyl Alkohol | | | | | 2,00 | |
| Natrium Carbomer | | | | | 0,30 | |
| Acrylates/C10-30 Alkyl Acrylate Crosspolymer | | | 0,30 | 0,40 | 0,10 | 0,10 |
| Ceteareth-20 | | | 1,00 | | | |
| Xanthan Gummi | 0,50 | | | 0,15 | | 0,50 |
| Dimethicon / Vinyl Dimethicon Crosspolymer | | | | 5,00 | | 3,00 |
| UVASorb® K2A | | | | | 3,50 | |
| Uvinul®A Plus | 0,25 | | | 0,50 | 2,00 | 1,50 |
| Butyl Methoxydibenzoylmethan | | | 3,50 | | | |
| Bis-Ethylhexyloxyphenol Methoxypheny Triazin | | 2,00 | | 0,25 | | |
| Terephthaliden Dicampher Sulfonsäure | | | | | | 0,50 |
| Dinatrium Phenyl Dibenzimidazol Tetrasulfonat | 0,75 | | | | | 1,00 |
| Phenylbenzimidazol Sulfonsäure | | | 2,00 | | | |
| Ethylhexyl Methoxycinnamat | | | 7,00 | | 5,00 | 8,00 |
| Methylen Bis-Benzotriazolyl Tetramethylbutylphenol | | | | | | |
| Butyl Methoxydibenzoylmethan | | 3,50 | | | | |
| Diethylhexyl Butamido Triazon | | | 2,00 | 2,00 | | |
| Ethylhexyl Triazon | 4,00 | 3,00 | | | 4,00 | |
| Octocrylen | | | | 10,00 | | 2,50 |
| Titandioxid MT-100 Z | 0,50 | 2,00 | 1,00 | 2,00 | 3,00 | 1,00 |
| C₁₂₋₁₅ Alkyl Benzoat | 2,00 | | 2,50 | | | |
| C18-36 Triglycerid Fettsäure | | | 1,00 | | | |
| Butylenglycol Dicaprylat/Dicaprat | 4,00 | | | | 6,00 | |
| Dicaprylyl Carbonat | | 3,00 | | | | |
| Dicaprylylether | | 2,00 | | | | |
| Cyclomethicon | | | | 7,50 | | |
| Lanolin | | | | | 0,35 | |
| **Hydrierte Sojaglyceride (Hydrogenated Vegetable Oil)** | 1,00 | 2,00 | 3,00 | 4,00 | 0,50 | 5,50 |
| PVP Hexadecen Copolymer | 0,50 | | 0,50 | | 0,50 | 1,00 |
| Ethylhexyloxyglycerin | | 0,75 | | 1,00 | | 0,50 |
| Glycerin | 10,00 | 5,00 | 5,00 | | 5,00 | 15,00 |
| Butylenglycol | | 7,00 | | | | |
| Glycin Soja | | | | 1,00 | | |
| Vitamin E Acetat | 0,50 | 0,25 | 0,50 | 0,25 | 0,75 | 1,00 |
| α - Glycosylrutin | | | | | 0,25 | |
| Trinatrium EDTA | | 1,00 | 1,00 | 0,10 | 0,20 | |
| lodopropinylbutylcarbamat | 0,20 | 0,10 | | | | 0,15 |
| Methylparaben | 0,50 | | 0,20 | | 0,15 | |
| Phenoxyethanol | 0,50 | 0,40 | 0,40 | | 1,00 | 0,60 |
| Ethanol | 3,00 | 10,00 | 4,00 | 3,50 | | 1,00 |
| Parfüm, Farbstoffe | q.s. | q.s. | q.s. | q.s. | q.s. | q.s. |
| Wasser | ad 100 | ad 100 | ad 100 | ad 100 | ad 100 | ad 100 |
| Neutralisationsmittel (Natriumhydroxid, Kaliumhydroxid) | qs | qs | qs | qs | qs | qs |
| pH-Wert | 4,5-5,5 | 5,0-7,0 | 5,0-7,0 | 5,0-7,0 | 4,0-6,0 | 5,0-7,5 |

| **6. Gelcremes** | | | | | |
|---|---|---|---|---|---|
| | **1** | **2** | **3** | **4** | **5** |
| Carbomer | 0,125 | 0,125 | 0,125 | 0,125 | 0,125 |
| Shea Butter | | | | 1,00 | |
| Mineralöl | | | | | 6,00 |
| Octyldodecanol | | | | 1,50 | |
| Caprylsäure/Caprinsäuretriglycerid | | | 4,00 | | |
| Dicaprylyl Carbonat | | 9,00 | | 1,00 | 3,00 |
| Dimethicon | | | | 0,50 | |
| Cyclomethicon | 9,00 | 2,00 | 3,00 | 2,00 | 1,00 |
| Diazolidinylharnstoff | 0,20 | 0,20 | 0,20 | 0,20 | 0,20 |
| Phenoxyethanol + Ethyl-, Methyl-, Propyl- Butyl-, Isobutylparaben | 0,50 | 0,50 | 0,50 | 0,50 | 0,50 |
| Parfum | | | | 0,25 | 0,25 |
| Glycerylstearatcitrat | 1,00 | 1,00 | 1,00 | 1,00 | 1,00 |
| Hydrierte Kokosfettsäureglycerid | 1,00 | 1,00 | 1,00 | | |
| **Hydrierte Sojaglyceride (Hydrogenated Vegetable Oil)** | 2,00 | 1,00 | 2,00 | 3,00 | 1,00 |
| Ammonium Acryloyldimethyltaurat/VP Copolymer | | | | | 0,125 |
| Hydroxyethylcellulose | 0,375 | 0,375 | 0,375 | 0,375 | 0,375 |
| Menthol | 0,10 | 0,50 | 1,00 | 0,10 | 0,10 |
| Wasser + Alcohol Denat | | | | 3,00 | |
| Wasser + Blue 1 | | 0,200 | 0,60 | 0,40 | |
| Wasser + Glycerin | 10,00 | 10,00 | 10,00 | 10,00 | 10,00 |
| Xanthangummi | 0,125 | 0,125 | 0,125 | 0,125 | |
| Wasser | ad 100,0 | ad 100,0 | ad 100,0 | ad 100,0 | ad 100,0 |
| Neutralisationsmittel (Natriumhydroxid, Kaliumhydroxid) | qs | qs | qs | qs | qs |
| pH-Wert | 4,5-5,5 | 6,5-8,5 | 5,0-7,0 | 4,0-6,0 | 5,0-7,5 |

| **7. Ölgele** | | | | | |
|---|---|---|---|---|---|
| | **1** | **2** | **3** | **4** | **5** |
| Octyldodecanol | 9,00 | 9,00 | | | |
| Caprylsäure/Caprinsäuretriglycerid | 9,00 | | 6,00 | | |
| C12-15- Alkyl Benzoate | | | | 5,00 | 8,00 |
| Butylenglycol Dicaprylat/Dicaprat | | 9,00 | | | 8,00 |
| Dicaprylylether | 9,00 | | | 4,00 | |
| Dicaprylylcarbonat | | 7,00 | | | |
| **Hydrierte Sojaglyceride (Hydrogenated Vegetable Oil)** | 5,00 | 7,00 | 3,00 | 1,50 | 7,00 |
| Ethyl Galaktomannan (N-Hance® AG 200) | 3,50 | | | | 4,00 |
| C20-40 Fettsäuren + Polyethylen (Performacid®350) | | | | 3,60 | |
| Hydroxyoctacosanyl Hydroxystearat | 2,00 | | | | |
| Disteardimonium Hectorit | 1,00 | | | | 1,00 |
| Cetyl Dimethicon | 0,50 | | 4,50 | | |
| Cyclomethicon | | | 15,00 | | 5,00 |
| UVASorb® K2A | | | | | 1,00 |
| Uvinul®A Plus | 1,00 | 3,50 | 2,75 | 2,00 | 0,50 |
| Ethylhexylmethoxycinnamat | 6,00 | | | 10,00 | 3,0 |
| Octocrylen | 3,50 | | 7,50 | 10,00 | |
| Ethylhexylsalicylat | | 3,50 | | | 4,00 |
| Ethylhexyl Triazon | | | 2,00 | | |
| Diethylhexyl Butamido Triazon | | 0,50 | | 3,00 | 4,0 |
| Polysaccharid-N-alkylurethanen, Inulincarbamat | 1,50 | 4,50 | 5,50 | 1,00 | 3,00 |
| Phenoxyethanol | 0,50 | | | | |
| Parfüm, Farbstoff | q.s. | q.s. | q.s. | q.s. | q.s. |
| Mineralöl | ad. 100 | ad. 100 | ad. 100 | | |
| Reisöl | | | | ad. 100 | ad. 100 |

| **8. Feststoffstablisierte Emulsionen** | | | | | |
|---|---|---|---|---|---|
| | **1** | **2** | **3** | **4** | **5** |
| Mineralöl | | | 16,00 | 16,00 | |
| Octyldodecanol | 9,00 | 9,00 | 5,00 | | |
| Caprylic/Capric Triglycerid | 9,00 | 9,00 | 6,00 | | |
| C12-15- Alkyl Benzoat | | | | 5,00 | 8,00 |
| Butylene Glykol Dicaprylat/Dicaprat | | | | | 8,00 |
| Dicaprylyl Ether | 9,00 | | | 4,00 | |
| Dicaprylyl Carbonat | | 9,00 | | | |
| Hydroxyoctacosanyl Hydroxystearat | 2,00 | 2,00 | 2,00 | 2,00 | 1,50 |
| Disteardimonium Hectorit | 1,00 | 0,750 | 0,50 | 0,50 | 0,25 |
| Cera Microcristallina + Paraffinöl | | | 2,50 | | 5,00 |
| **Hydrierte Sojaglyceride (Hydrogenated Vegetable Oil)** | 2,50 | 0,75 | 1,00 | 3,50 | 1,00 |
| Hydroxypropyl Methylcellulose | 0,15 | | | | 0,05 |
| Dimethicon | | | 4,50 | | |
| UVASorb® K2A | 2,00 | 5,00 | 3,00 | 1,50 | 1,00 |
| Bis-Ethylhexyloxyphenol Methoxypheny Triazin | 1,00 | 3,00 | 0,75 | 1,00 | 1,00 |
| Terephthaliden Dicampher Sulfonsäure | | 2,00 | | | 0,50 |
| Phenylbenzmidazol Sulfonsäure | 2,00 | 0,50 | 1,00 | | |
| Uvinul® A Plus | | | 2,75 | | 0,50 |
| Ethylhexylmethoxycinnamat | 6,00 | | | | 3,0 |
| Octocrylen | 3,50 | | 7,50 | | |
| Ethylhexyl Salicylat | | 3,50 | | | 4,00 |
| Diethylhexyl Butamido Triazon | | | | | 4,0 |
| Titandioxid Eusolex ® T-2000 | | 2,00 | 4,00 | 2,00 | 4,00 |
| Titandioxid T 805 ® | | | | | 3,00 |
| Silica Dimethyl Silylat | | | 1,00 | | |
| Bornitrid | 2,00 | | | 3,00 | |
| Tapioca Stärke | | | | 1,00 | |
| Natriumchlorid | 1,00 | 1,00 | 1,00 | 1,00 | |
| Glycerin | 5,0 | 10,0 | | 6,00 | 10,0 |
| Trinatrium EDTA | | 1,00 | | 1,00 | |
| Methylparaben | 0,21 | | | | 0,20 |
| Propylparaben | 0,07 | | | | |
| Phenoxyethanol | 0,50 | | 0,40 | 0,40 | 0,50 |
| Hexamidin Diisethionat | | | | | 0,08 |
| Diazolidinyl Harnstoff | | | 0,28 | 0,28 | |
| Alkohol | | 5,00 | | 2,50 | |
| Parfüm | 0,45 | 0,20 | | | 0,45 |
| Wasser | ad 100 | ad 100 | ad 100 | ad 100 | ad 100 |

### Gele:

| Beispiel 1 (Eye Shadow Gel): | |
|---|---|
| | Gew.-% |
| PEG-8 (Polyethylenglycol 400) | 2,00 |
| Ethanol | 5,00 |
| Aristoflex AVC | 1,50 |
| Glycerin | 2,00 |
| Panthenol | 0,50 |
| Tocopherolacetat | 0,50 |
| Timiron Splendid blue ® (Merck KgaA) | 4,50 |
| Chromoxid grün | 1,00 |
| **Hydrierte Sojaglyceride (Hydrogenated Vegetable Oil)** | **1,00** |
| Parfüm, Konservierungsmittel, NaOH, Komplexbildner, Farbstoffe, Antioxidantien etc. | q.s. |
| Wasser | ad 100,00 |

| Beispiel 2 (Highlighter Gel): | |
|---|---|
| | Gew. % |
| Carbomer | 1,50 |
| Glycerin | 2,50 |
| 1,3 Butylenglycol | 2,50 |
| Glitzerpigmente (z.B. Helicone HC Scarabeus, Wacker) | 1,00 |
| EDTA | 0,20 |
| Dimethicone | 1,50 |
| **Hydrierte Sojaglyceride (Hydrogenated Vegetable Oil)** | **2,00** |
| Parfüm, Konservierungsmittel, NaOH, Farbstoffe, Antioxidantien, etc. | q.s. |
| Wasser | ad 100,00 |

| Beispiel 3 (Eye Liner Gel): | |
|---|---|
| | Gew. % |
| Perlglanzpigmente | 10,00 |
| Eisenoxid | 3,00 |
| Silica | 2,00 |
| Aristoflex AVC | 1,00 |
| Hyroxyproylethyl Cellulose | 0,35 |
| **Hydrierte Sojaglyceride (Hydrogenated Vegetable Oil)** | **5,00** |
| Citric Acid | q.s. |
| Glycerin | 5,00 |
| PVP / VA Copolymer | 2,00 |
| Parfüm, Konservierungsmittel, Farbstoffe, NaOH, Komplexbildner, Antioxidantien, etc. | q.s. |
| Wasser | ad 100,00 |

### Make-up

| Beispiel 1 (Emulsions-Make-up): | |
|---|---|
| | Gew. % |
| PEG-30 Stearat | 2,00 |
| Glycerinmonostearat | 1,00 |
| Stearinsäure | 1,00 |
| Cyclomethicon | 7,00 |
| Octyldodecanol | 7,00 |
| Isopropyl Lanolat | 4,00 |
| Squalan | 2,00 |
| **Hydrierte Sojaglyceride (Hydrogenated Vegetable Oil)** | **3,00** |
| Octyl Methoxycinnamat | 2,00 |
| Butyl Methoxydibenzoylmethan | 1,00 |
| Xanthan Gum | 0,20 |
| Glycerin | 5,00 |
| Butylenglkcol | 2,00 |
| Vitamin E Acetat | 1,00 |
| Magnesiumsilikat | 1,00 |
| Glimmer | 1,00 |
| Eisenoxid | 1,00 |
| Titandioxid | 2,50 |
| Talkum | 5,00 |
| EDTA | 0,50 |
| Parfüm, Konservierungsmittel, NaOH, Antioxidantien etc. | q.s. |
| Wasser | ad 100,00 |

| Beispiel 2 (Emulsions-Make-up): | |
|---|---|
| | Gew. % |
| Cyclomethicon and PEG /PPG -18/18 Dimethicon | |
| (z.B. Dow Corning 3225 Formulation Aid) | 10,00 |
| Cyclomethicon | 10,00 |
| Bienenwachs | 3,00 |
| Polyglyceryl-4 Oleat | 2,00 |
| Eisenoxid | 1,00 |
| Titandioxid | 2,50 |
| Talkum | 12,00 |
| Natrium Chlorid | 2,00 |
| **Hydrierte Sojaglyceride (Hydrogenated Vegetable Oil)** | **5,00** |
| Parfüm, Konservierungsmittel, NaOH, Antioxidantien etc. | q.s. |
| Wasser | ad 100,00 |

| Beispiel 3 (Cover Cream): | |
|---|---|
| | Gew. % |
| Cyclomethicon | 44,00 |
| Bienenwachs | 3,00 |
| Carnaubawachs | 5,00 |
| Lanolinöl | 5,00 |
| Paraffinöl | 8,40 |
| **Hydrierte Sojaglyceride (Hydrogenated Vegetable Oil)** | **7,00** |
| Cetyl Alkohol | 2,60 |
| Eisenoxid | 3,00 |
| Titandioxid | 7,50 |
| Nylon | 6,00 |
| Talkum | 10,50 |
| Parfüm, Konservierungsmittel, Antioxidantien etc. | q.s. |

| Beispiel 4 (Emulsions-Make-up): | |
|---|---|
| | Gew. % |
| Cyclomethicon | 18,00 |
| Phenyltrimethicon | 3,00 |
| Cetyl PEG/PPG - 10/1 Dimethicon (z.B. Abil EM 90) | 4,00 |
| Paraffinöl | 3,00 |
| Eisenoxid | 2,30 |
| Titandioxid | 4,50 |
| Talkum | 2,00 |
| Natrium Chlorid | 2,00 |
| Quaternium-18 Hectorit | 0,30 |
| Propylen Carbonat | 0,08 |
| **Hydrierte Sojaglyceride (Hydrogenated Vegetable Oil)** | **5,00** |
| Parfüm, Konservierungsmittel, NaOH, Antioxidantien etc. | q.s. |
| Wasser | ad 100,00 |

## Patentansprüche

1. Verwendung von hydrierten Sojaglyceriden in kosmetischen Zubereitungen zur Behandlung und Prophylaxe der Symptome der Hautalterung.

2. Verwendung von hydrierten Sojaglyceriden in kosmetischen Zubereitungen zur Straffung und Glättung der Haut sowie zur Reduzierung von Falten.

3. Verwendung von hydrierten Sojaglyceriden in kosmetischen Zubereitungen zur Befeuchtung der Haut.

4. Verwendung von hydrierten Sojaglyceriden in kosmetischen Zubereitungen zur Erhöhung der Hautelastizität und Weichheit der Haut.

5. Verwendung von hydrierten Sojaglyceriden in kosmetischen Zubereitungen zur Regulierung des Fettgehaltes der Haut.

6. Verwendung von hydrierten Sojaglyceriden in kosmetischen Zubereitungen zur Erhöhung der Wirksamkeit kosmetischer Wirkstoffe.

7. Verwendung nach einem der vorhergehenden Ansprüche, **dadurch gekennzeichnet, dass** die Zubereitung in Form einer Emulsion vorliegt.

8. Verwendung nach Anspruch 7, **dadurch gekennzeichnet, dass** die Zubereitung ein oder mehrere Polyole enthält.

9. Verwendung nach Anspruch 7 oder 8, **dadurch gekennzeichnet, dass** die Zubereitung ein oder mehrere Wachse mit einem Schmelzpunkt von größer 30 °C enthält.

10. Verwendung nach einem der vorhergehenden Ansprüche, **dadurch gekennzeichnet, dass** die Zubereitung hydrierte Sojaglyceride in einer Konzentration von 0,5 bis 5 Gewichts-%, bezogen auf das Gesamtgewicht der Zubereitung enthält.
